(19) Europäisches Patentamt
European Patent Office
Office européen des brevets

(11) Publication number: **0 423 520 B1**

(12) # EUROPEAN PATENT SPECIFICATION

(45) Date of publication of patent specification: **02.08.95**

(51) Int. Cl.6: **C09K 19/30**, C09K 19/02

(21) Application number: **90118589.2**

(22) Date of filing: **27.09.90**

(54) **Liquid crystalline composition for the use in a twisted nematic cell.**

(30) Priority: **19.10.89 GB 8923579**
**19.10.89 GB 8923578**

(43) Date of publication of application:
**24.04.91 Bulletin 91/17**

(45) Publication of the grant of the patent:
**02.08.95 Bulletin 95/31**

(84) Designated Contracting States:
**AT BE CH DE DK ES FR GB GR IT LI LU NL SE**

(56) References cited:
**EP-A- 0 104 327**
**EP-A- 0 344 557**

(73) Proprietor: **MERCK PATENT GmbH**
**Postfach,**
**Frankfurter Strasse 250**
**D-64271 Darmstadt (DE)**

(72) Inventor: **Coates, David, Dr.**
**87, Sopwith Crescent**
**Merley Wimborne,**
**Dorset BH21 3SW (GB)**
Inventor: **Clemitson, Robert William, Dr.**
**2 Plumer Road**
**Waterloo Estate,**
**Broadstone (GB)**
Inventor: **Krause, Joachim, Dr.**
**Samuel-Morse-Strasse 14**
**D-6110 Dieburg (DE)**
Inventor: **Kurmeier, Hans-Adolf, Dr.**
**Hinter der Schule 3a**
**D-6104 Seeheim-Jugenheim (DE)**
Inventor: **Dorsch, Dieter, Dr.**
**Königsbergerstrasse 17a**
**D-6105 Ober-Ramstadt (DE)**
Inventor: **Kompter, Hans-Michael, Dr.**
**Mainzer Strasse 14**
**D-6108 Weiterstadt (DE)**
Inventor: **Geelhaar, Thomas, Dr.**
**Trajanstrasse 12**
**D-6500 Mainz (DE)**
Inventor: **Poetsch, Eike, Dr.**
**Am Buchwald 4**
**D-6109 Mühltal 6 (DE)**
Inventor: **Bartmann, Ekkehard, Dr.**
**Dieburger Weg 12a**
**D-6106 Erzhausen (DE)**
Inventor: **Pauluth, Detlef, Dr.**
**Königsberger Strasse 17**
**D-6105 Ober-Ramstadt (DE)**
Inventor: **Hittich, Reinhard, Dr.**
**Am Kirchberg 11**
**D-6101 Modautal 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 423 520 B1

## Description

The invention relates to nematic liquid crystalline compositions comprising a group (A) with one or more components exhibiting a positive dielectric anisotropy ($\Delta\epsilon$) and a group (B) with one or more components exhibiting a neutral dielectric anisotropy characterized in that

(a) at least one component of group (A) is a compound of the formula I

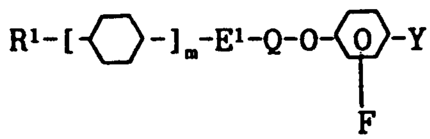

(I)

wherein

- $R^1$ is an alkyl or alkenyl residue each with up to 16 C atoms, wherein one or two non-adjacent $CH_2$-groups of these residues may be replaced by -O-, -S-, -CO-O-, or -O-CO-,
- $E^1$ is $-CH_2CH_2-$ or -CH = CH-,
- Q is -CO- or $-CH_2-$,
- Y is halogen or CN, and
- m is 1 or 2

or a compound of the formula II

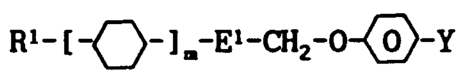

(II)

wherein $R^1$, $E^1$, Y and m have the meaning given for formula I,
or a compound of the formula III

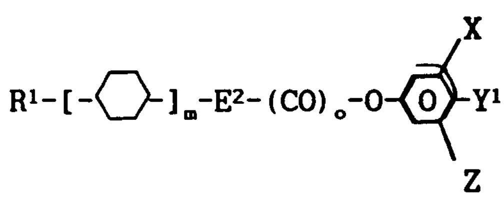

(III)

wherein

- $R^1$ is an alkyl or alkenyl residue each with up to 16 C atoms, wherein one or two non-adjacent $CH_2$-groups of these residues may be replaced by -O-, -S-, -CO-O-, or -O-CO,
- $E^2$ is $-(CH_2)_n-$ or $-CH=CH-(CH_2)_{n-2}-$
- X and Z are each independently H or F,
- $Y^1$ is F, Cl, NCS, $CF_3$, $OCF_3$ or $OCF_2H$,
- m is 1 or 2,
- n is 1, 2 or 3,
- o is 0 or 1 and

with the proviso that m is 2 or n is 2 or 3 in the case that $Y^1$ is F, Cl or NCS,
or a compound of the formula IV

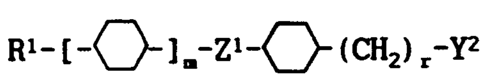

(IV)

wherein $R^1$ and m have the meaning given for formula I, and

$Z^1$ is $-(CH_2)_4-$, $-(CH_2)_3-O-$ or $-CH=CH-CH_2CH_2-$

$Y^2$ is $CF_3$, $OCF_3$ or $-CH=CH-CF_3$, and

r is 0 to 7,

or that

(b) at least one component of group (B) is a compound of the formula V

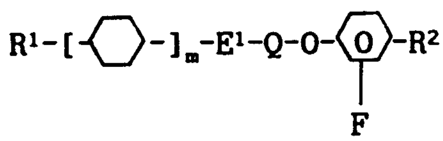

(V)

wherein $R^1$, $E^1$, Q and m have the meaning given for formula I, and

$R^2$ is an alkyl or alkyl residue with up to 16 C atoms wherein one or two non-adjacent $CH_2$ groups of these residues may be replaced by -O-, -S-, -CO-O- or -O-CO-

or a compound of the formula VI

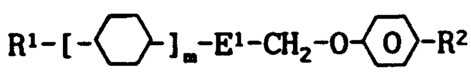

VI

wherein $R^1$, $R^2$, $E^1$ and m have the meaning given for formula V.

The invention was based on the object of discovering that new liquid crystalline composition with advantageous values for optical and dielectric anisotropy for the use in twisted nematic cells can be obtained by admixing a group (A) with one or more components exhibiting a positive dielectric anisotropy and a group (B) with one or more components exhibiting a neutral dielectric anisotropy wherein at least one component is a compound of the formulae I, II, III, IV (group A) or V, VI (group B). The compounds of the formulae I and III are embraced by the broad general formula of DE-OS-32 25 290 but neither compounds with laterally fluorine substituents are disclosed there nor is described how to prepare them.

The compounds of the formulae II and VI are embraced by the broad general formula of DE-OS 34 08 882. But none of them describes the synthesis of the laterally fluorinated compounds of the formulae II and IV.

The non-fluorinated 4-substitued phenyl esters of 3-(4-alkylcyclohexyl)-propionic acid are described by DD-OS-155-078 and 155 063.

Similar compounds are known from the Japanese Patent Applications J 60215652 and J 61010553 which claim 2-phenylethylesters of 4-alkylcyclohexylene carboxylic acids and from the Japanese Patent Application J 61100548 which claims 4-substituted benzyl 4-alkylcyclohexylacetates.

Similar compounds with a $O-CF_3$ wing group are known from International Patent Application WO 89-02884.

4-(trans-4-Alkylcyclohexylmethoxy)-fluorobenzenes and 4-(trans,trans-4'-alkylbicyclohexyl-4-yl-methoxy)-3-fluorobenzonitriles are known from Japanese Patent Application J 60013731.

EP-A-0344577 which is prior art under Article 54 (3) EPC discloses compounds of formulae

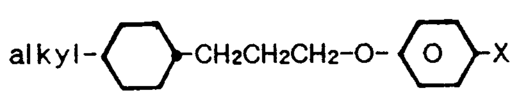

with X being F, Cl or -NCS, and

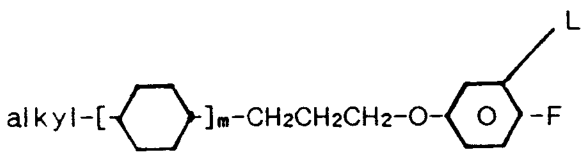

with m being 1 or 2 and $L^1$ being H or F, which are excluded from claims 10 and 11 by the proviso clauses a), b) and c).

It has now been found that compounds of formulae I, II, III, IV, V and VI are highly suitable as components of liquid crystalline compositions for the use in twisted nematic cells. In particular, they have especially advantageous values of optical and dielectric anisotropy. It is also possible to obtain stable liquid crystalline phases with a broad nematic mesophase range and a comparatively low viscosity with the aid of these compounds.

Depending on the choice of the substituents, the compounds of the formulae I, II, III, IV, V and VI can be used as the base materials from which liquid crystal media are predominantly composed; however, it is also possible for compounds of the formulae I, II, III, IV, V and VI to be added to liquid crystal base materials of other classes of compounds, for example in order to influence the dielectric and/or optical anisotropy and/or the viscosity and/or the mesophase range of such a dielectric.

The compounds of the formulae I, II, III, IV, V and VI are colourless in the pure state and are liquid crystalline in a temperature range which is favourably placed for electrooptical use. They are very stable towards chemicals, heat and light.

The invention thus relates to a liquid crystalline composition for the use in a twisted nematic cell being an admixture of a group (A) with one or more components exhibiting a positive dielectric anisotropy ($\Delta\epsilon$) and a group (B) with one or more components exhibiting a neutral dielectric anisotropy characterized in that

(a) at least one component of group (A) is a compound of the formula I, II, III or IV or

(b) at least one component of group (B) is a compound of the formula V or VI.

In particular to liquid crystalline compositions wherein the dielectric anisotropy ($\Delta\epsilon$) of the group A is greater than +6 or wherein

the dielectric anisotropy ($\Delta\epsilon$) of the group B is -4 to +3.

Furthermore, the invention relates to a twisted nematic cell containing a liquid crystalline composition according to the invention in particular to a cell in which the twist angle is between 160 and 300°.

Some of the compounds of the formulae I, II, III, IV, V and VI are known, but some are still novel.

Therefore, the invention relates to the novel compounds of the formulae I, II, III and IV, in particular to the compounds of the formulae III and IV.

The compounds of the formulae I, II, V and VI, wherein $E^1$ is $-CH_2CH_2-$ are particularly preferred.

The compounds of the formula III wherein $E^2$ is $-(CH_2)_n-$ with n being 1, 2 or 3 are particularly preferred.

Above and below, $R^1$, $R^2$, Y, X, $Y^1$, Z, $Y^2$, $Z^1$, Q, m, n, o and r have the meaning given unless expressly indicated otherwise.

The laterally fluorinated compounds with a positive dielectric anisotropie of the formula I include compounds with two and three rings of the formulae Ia to Ih

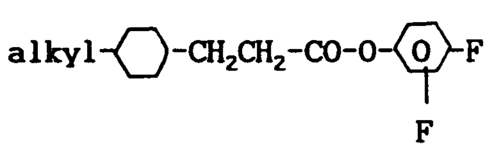

Ia

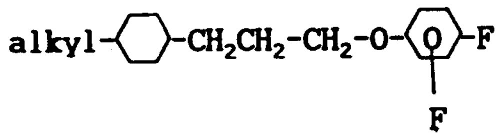
Ib

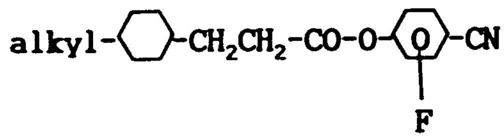
Ic

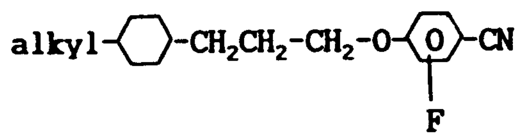
Id

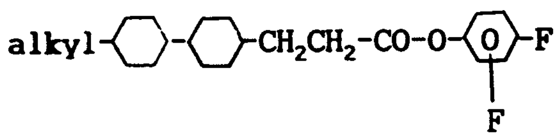
Ie

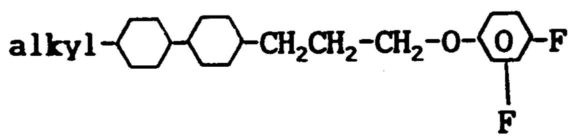
If

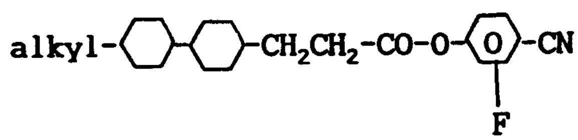
Ig

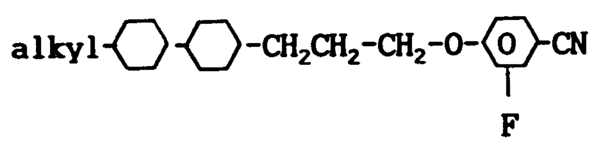
Ih

wherein alkyl denotes an alkyl residue with up to 16 C atoms.

The non-fluorinated 3-cyclohexylpropylethers of the formula II include compounds with two and three rings of the formulae IIa to IId

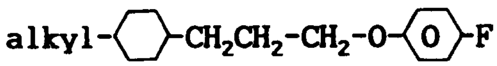

IIa

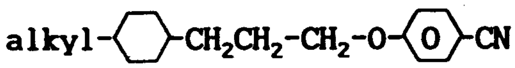

IIb

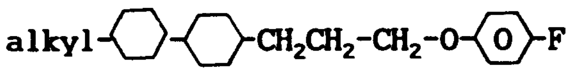

IIc

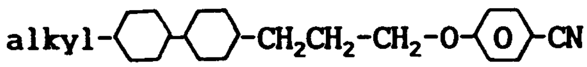

IId

The compounds of the formulae III include compounds with two rings of the formulae IIIa to IIIl:

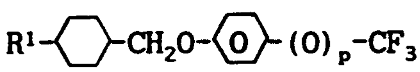

IIIa

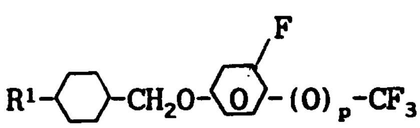

IIIb

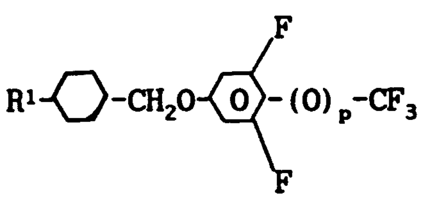

IIIc

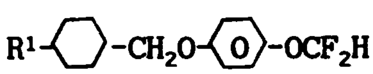

IIId

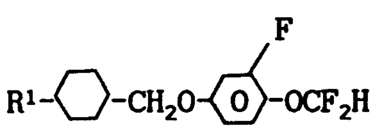

IIIe

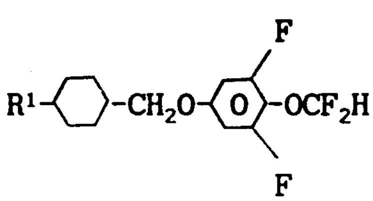

IIIf

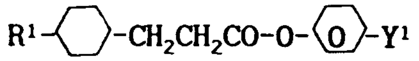

IIIg

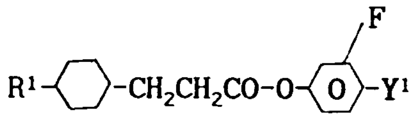

IIIh

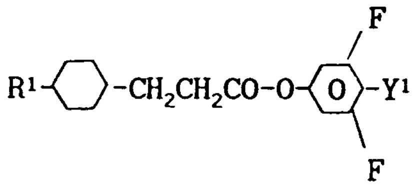

IIIi

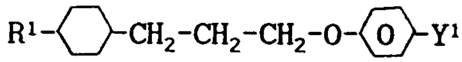

IIIj

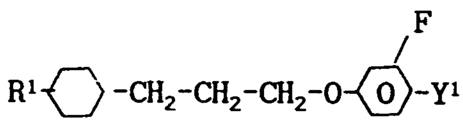

IIIk

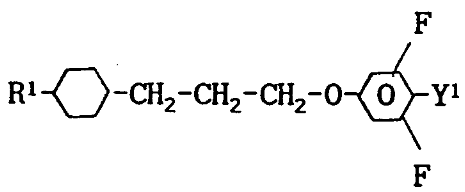

IIIl

wherein $R^1$ and $Y^1$ have the meaning given for formula III of claim 1, and p is 0 or 1, with the proviso that

a) the compounds of formula IIIj, wherein $Y^1$ is F, Cl or NCS, are excluded, and

b) the compounds of formula IIIk, wherein $Y^1$ is F are excluded.

and compounds with three rings of formulae IIIm to IIIu:

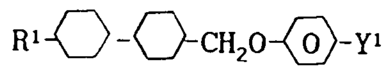 IIIm

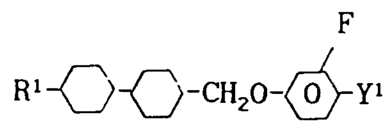 IIIn

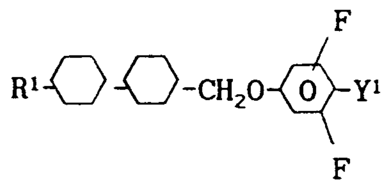 IIIo

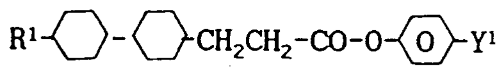 IIIp

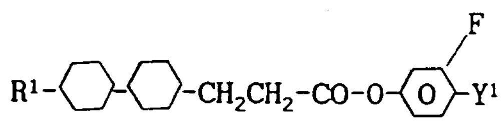 IIIq

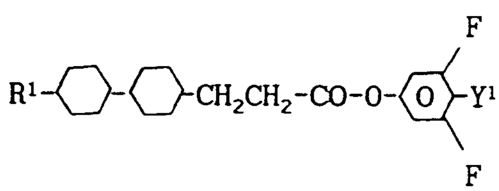 IIIr

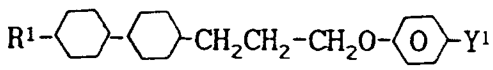 IIIs

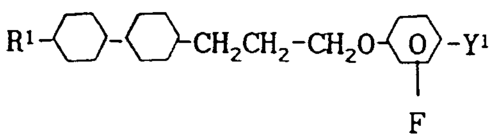 IIIt

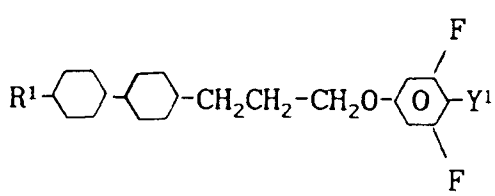 IIIu

wherein
$R^1$ and $Y^1$ have the meaning given for formula III of claim 1,
with the proviso that

c) the compounds of formula IIIs and IIIt, wherein $Y^1$ is F, are excluded.

Furthermore, the invention relates to a liquid crystalline medium being a mixture of at least two compounds, characterized in that at least one compound is a cyclohexane derivative of the formula III.

In the compounds of the part formulae IIIg to IIIu $Y^1$ denotes preferably F, Cl, $CF_3$ or Op-$CF_3$, in particular or O-$CF_3$.

The compounds of the formulae IV include the preferred compounds of the formulae IVa to IVi with two rings:

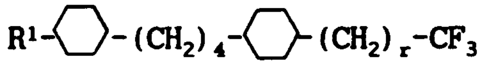 IVa

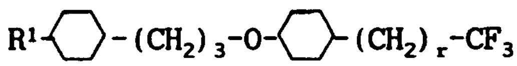 IVb

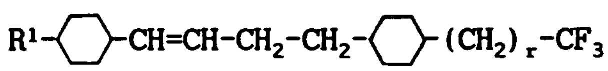 IVc

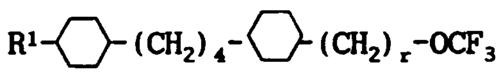 IVd

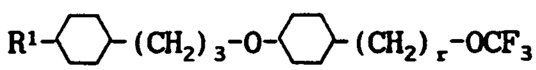 IVe

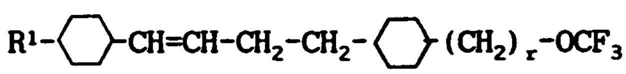 IVf

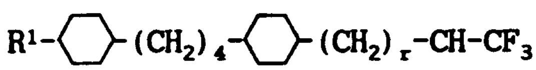 IVg

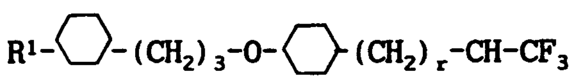 IVh

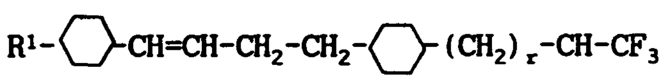 IVi

and with three rings of the formulae IVj to IVv:

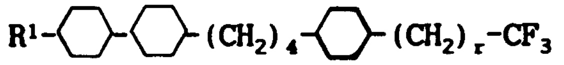 IVj

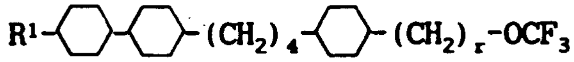 IVk

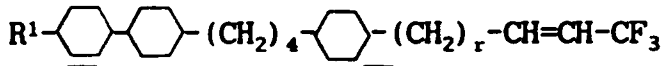 IVl

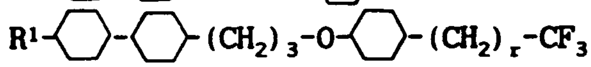 IVm

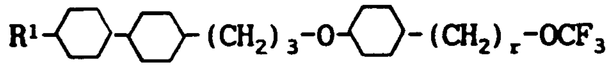 IVn

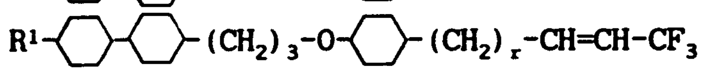 IVo

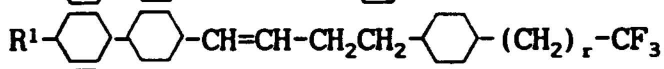 IVp

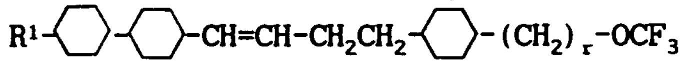 IVq

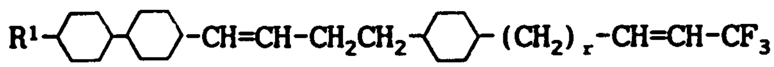 IVr

The compounds of the formulae IV, wherein r is 0, 1, 2 or 3 are preferred in particular wherein r is 0 or 1. Furthermore the invention relates to a liquid crystalline medium being a mixture of at least two components characterized in that at least one component is a compound of the formula IV.

The laterally fluorinated compounds with a neutral dielectric anisotropie fo the formulae V include compounds with two and three rings of the formulae Va to Vd

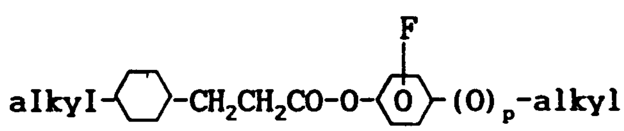 Va

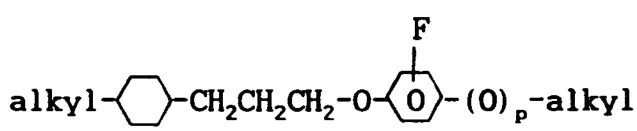 Vb

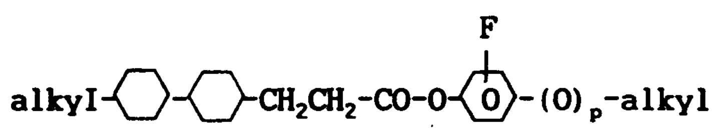 Vc

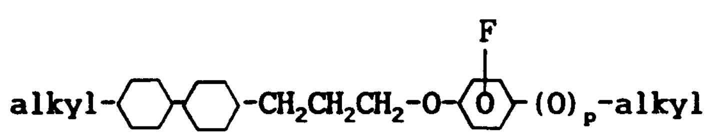 Vd

wherein p is 0 or 1.

The neutral non-fluorinated 3-cyclohexylpropylethers of the formula VI include compounds with two and three rings of the formulae VIa and VIb.

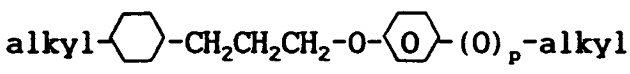 VIa

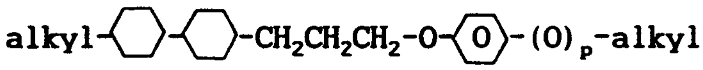 VIb

wherein p is 0 or 1.

Laterally fluorinated compounds, however, are especially preferred because they generally exhibit a comperatively low viscosity.

$R^1$ and/or $R^2$ are independently from each other alkyl, alkoxy, oxaalkyl, thioalkyl, alkorycarbonyl, alkanoyloxy or alkenyl and both can exhibit a straight-chain or branched structure.

The compounds of the formulae IV, V and VI wherein the wing groups R1 and R2 each have more than 4-C atoms are particularly preferred as components of chiral tilted smectic liquid crystalline compositions.

Alkyl or alkoxy preferably are straight-chain and have 2, 3, 4, 5, 6 or 7 C atoms. Accordingly they are preferably ethyl, propyl, butyl, pentyl, hexyl, heptyl, ethoxy, propoxy, butoxy, pentoxy, hexoxy or heptoxy, also methyl, octyl, nonyl, decyl, undecyl, dodecyl, tridecyl, tetradecyl, pentadecyl, methoxy, octoxy, nonoxy, decoxy, undecoxy, dodecoxy, tridecoxy or tetradecoxy.

Oxaalkyl is preferably straight-chain 2-oxapropyl (= methoxy~ methyl), 2-(= ethoxymethyl) or 3-oxybutyl (= 2-methoxyethyl), 2-, 3- or 4-oxapentyl, 2-, 3-, 4- or 5-oxahexyl, 2-, 3-, 4- 5- or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-, 7-, 8-, or 9-oxadecyl.

Alkenyl is preferably straight-chain and has 2 or 10 C atoms. It is accordingly, in particular, vinyl, prop-1- or prop-2-enyl, but-1-, -2- or -3-enyl, pent-1-, -2-, -3- or -4-enyl, hex-1-, -2-, -3-, -4- or -5-enyl, hept-1-, -2-, -3-, -4-, -5- or -6-enyl, oct-1-, -2-, -3-, -4-, -5- -6- or -7-enyl, non-1-, -2-, -3-, -4-, -5-, -6-, -7- or -8-enyl or dec-1-, -2-, -3-, -4-, -5-, -6-, -7-, -8- or -9-enyl.

Compounds of the formulae I, II, III and IV containing a branched terminal group can occasionally be of importance because of an improved solubility in the customary liquid crystal base materials. Compounds of the formulae I and II with a branched terminal group $R^1$ can be used as chiral doping substances if they are optically active. Smectic compounds of this type are suitable as components of ferroelectric materials.

Branched groups of this type as a rule contain not more than one chain branching. Preferred branched radicals are isopropyl, 2-butyl (= 1-methylpropyl), isobutyl (= 2-methylpropyl, 2-methylbutyl, isopentyl, (= 3-methylbutyl), 2-methylpentyl, 2-ethylhexyl, 2-propylpentyl, 2-octyl, isopropoxy, 2-methyl propoxy, 2-methylbutoxy, 3-methylbutoxy, 2-methylpentoxy, 3-methylpentoxy, 2-ethylhexoxy, 2-methylhexoxy, 1-methylhexoxy, 1-methylheptoxy (= 2-octyloxy), 2-oxa-3-methylbutyl, 3-oxa-4-methylpentyl, 4-methylhexyl, 2-nonyl, 2-decyl, 2-dodecyl, 6-methyloctoxy, 6-methyloctanoyloxy, 4-methylheptyloxycarbonyl, 2-methylbutyryloxy, 3-methylvaleryloxy, 4-methylhexanoyloxy, 2-methyl-3-oxapentyl and 2-methyl-3-oxahexyl.

In the case of compounds with branched terminal group, formulae I, II, III, IV, V and VI include both the optical antipodes and racemates as well as mixtures thereof.

In the following smaller groups A to K of preferred compounds of the formula III and the part formulae thereof alkyl denotes preferably normal alkyl group with 2 to 8 C atoms, Cyc is a 1,4-cyclohexylene group, Phe is a 1,4-phenylene group, PheF is a group of the formula

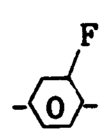

and PhFF a group of the formula

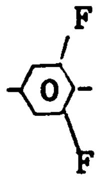

:

A)
alkyl-Cyc-$CH_2O$-Phe-$OCF_3$
alkyl-Cyc-$CH_2O$-Phe-$CF_3$
alkyl-Cyc-$CH_2O$-Phe-$OCF_2H$
alkyl-Cyc-$CH_2O$-PheF-$OCF_3$
alkyl-Cyc-$CH_2O$-PheF-$CF_3$
alkyl-Cyc-$CH_2O$-PheF-$OCF_2H$
alkyl-Cyc-$CH_2O$-PhFF-$OCF_3$
alkyl-Cyc-$CH_2O$-PhFF-$CF_3$
alkyl-Cyc-$CH_2O$-PhFF-$OCF_2H$
B)
alkyl-Cyc-$CH_2CH_2$-CO-O-Phe-$OCF_3$
alkyl-Cyc-$CH_2CH_2$-CO-O-Phe-$CF_3$
alkyl-Cyc-$CH_2CH_2$-CO-O-Phe-$OCF_2H$
alkyl-Cyc-$CH_2CH_2$-CO-O-Phe-F
alkyl-Cyc-$CH_2CH_2$-CO-O-Phe-NCF
alkyl-Cyc-$CH_2CH_2$-CO-O-Phe-Cl
C)
alkyl-Cyc-$CH_2CH_2$-COO-PheF-$OCF_3$
alkyl-Cyc-$CH_2CH_2$-COO-PheF-$CF_3$
alkyl-Cyc-$CH_2CH_2$-COO-PheF-$OCF_2H$
alkyl-Cyc-$CH_2CH_2$-COO-PheF-F
alkyl-Cyc-$CH_2CH_2$-COO-PheF-NCS
alkyl-Cyc-$CH_2CH_2$-COO-PheF-Cl
D)
alkyl-Cyc-$CH_2CH_2$-COO-PhFF-$OCF_3$
alkyl-Cyc-$CH_2CH_2$-COO-PhFF-$CF_3$
alkyl-Cyc-$CH_2CH_2$-COO-PhFF-$OCF_2H$
alkyl-Cyc-$CH_2CH_2$-COO-PhFF-F
alkyl-Cyc-$CH_2CH_2$-COO-PhFF-NCS
alkyl-Cyc-$CH_2CH_2$-COO-PhFF-Cl
E)
alkyl-Cyc-$CH_2CH_2CH_2$-O-Phe-$OCF_3$
alkyl-Cyc-$CH_2CH_2CH_2$-O-Phe-$CF_3$
alkyl-Cyc-$CH_2CH_2CH_2$-O-Phe-F
alkyl-Cyc-$CH_2CH_2CH_2$-O-Phe-NCS
alkyl-Cyc-$CH_2CH_2CH_2$-O-Phe-Cl
F)
alkyl-Cyc-$CH_2CH_2CH_2$-O-PheF-$OCF_3$
alkyl -Cyc-$CH_2CH_2CH_2$-O-PheF-$CF_3$
alkyl-Cyc-$CH_2CH_2CH_2$-O-PheF-F
alkyl-Cyc-$CH_2CH_2CH_2$-O-PheF-NCS
alkyl-Cyc-$CH_2CH_2CH_2$-O-PheF-Cl
G)
alkyl-Cyc-$CH_2CH_2CH_2$-O-PhFF-$OCF_3$
alkyl-Cyc-$CH_2CH_2CH_2$-O-PhFF-$CF_3$
alkyl-Cyc-$CH_2CH_2CH_2$-O-PhFF-F
alkyl-Cyc-$CH_2CH_2CH_2$-O-PhFF-NCS

alkyl-Cyc-$CH_2CH_2CH_2$-O-PhFF-Cl

H)

alkyl-Cyc-Cyc-$CH_2O$-Phe-$OCF_3$

alkyl-Cyc-Cyc-$CH_2O$-Phe-$CF_3$

alkyl-Cyc-Cyc-$CH_2O$-Phe-F

alkyl-Cyc-Cyc-$CH_2O$-PheF-$OCF_3$

alkyl-Cyc-Cyc-$CH_2O$-PheF-$CF_3$

alkyl-Cyc-Cyc-$CH_2O$-PheF-F

alkyl-Cyc-Cyc-$CH_2O$-PheF-NCS

alkyl-Cyc-Cyc-$CH_2O$-PheF-Cl

alkyl-Cyc-Cyc-$CH_2O$-PhFF-$OCF_3$

alkyl-Cyc-Cyc-$CH_2O$-PhFF-$CF_3$

alkyl-Cyc-Cyc-$CH_2O$-PhFF-F

alkyl-Cyc-Cyc-$CH_2O$-PhFF-Cl

alkyl-Cyc-Cyc-$CH_2O$-PhFF-NCS

I)

alkyl-Cyc-Cyc-$CH_2CH_2$-CO-O-Phe-$OCF_3$

alkyl-Cyc-Cyc-$CH_2CH_2$-CO-O-Phe-$CF_3$

alkyl-Cyc-Cyc-$CH_2CH_2$-CO-O-Phe-F

alkyl-Cyc-Cyc-$CH_2CH_2$-CO-O-Phe-Cl

alkyl-Cyc-Cyc-$CH_2CH_2$-CO-O-Phe-NCS

J)

alkyl-Cyc-Cyc-$CH_2CH_2$-CO-O-PheF-$OCF_3$

alkyl-Cyc-Cyc-$CH_2CH_2$-CO-O-PheF-$CF_3$

alkyl-Cyc-Cyc-$CH_2CH_2$-CO-O-PheF-F

alkyl-Cyc-Cyc-$CH_2CH_2$-CO-O-PheF-Cl

alkyl-Cyc-Cyc-$CH_2CH_2$-CO-O-PheF-NCS

K)

alkyl-Cyc-Cyc-$CH_2CH_2$-CO-O-PhFF-$OCF_3$

alkyl-Cyc-Cyc-$CH_2CH_2$-CO-O-PhFF-$CF_3$

alkyl-Cyc-Cyc-$CH_2CH_2$-CO-O-PhFF-F

alkyl-Cyc-Cyc-$CH_2CH_2$-CO-O-PhFF-Cl

alkyl-Cyc-Cyc-$CH_2CH_2$-CO-O-PhFF-NCS

The compounds of the formulae I to VI are prepared by methods which are known per se, such as are described in the literature (for example in the standard works, such as Houben-Weyl, Methoden der Organischen Chemie [Methods of Organic Chemistry], Georg-Thieme-Verlag, Stuttgart), and in particular under reaction conditions which are known and suitable for the reactions mentioned in more detail here can also be used in this connection.

If desired, the starting substances can also be formed in situ, such that they are not isolated from the reaction mixture but are immediately reacted further to give the compounds of the formulae I to VI.

Esters of the formulae I to V can be obtained by esterification of corresponding carboxylic acids (or their reactive derivatives) with alcohols or phenols (or their reactive derivatives) preferably the corresponding carboxylic acid and the alcohol or phenol are reacted with water absorbing means as, for example, mol sieves or carbodiimides, particularly preferably with dicyclohexylcarbodiimide .

The corresponding carboxylic acids of the formula VIIa and phenols of the formula VIIIa to VIIIe are known or can be prepared by processes analogous to known processes.

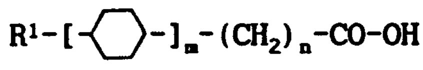

VIIa

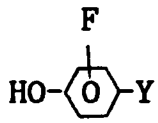

VIIIa

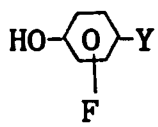

VIIIb

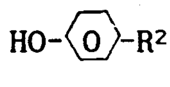

VIIIc

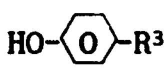

VIIId

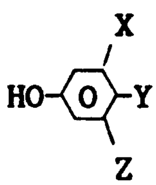

VIIIe

Particularly suitable reactive derivatives of the carboxylic acids mentioned are the acid halides, above all the chlorides and bromides, and furthermore the anhydrides, for example also mixed anhydrides, preferably those of the corresponding carboxylic acids and trifluoroacetic acid formed in situ by mixing these carboxylic acids with trifluoroacetic anhydride, azides or esters, in particular alkyl esters with 1-4 C atoms in the alkyl group.

Possible reactive derivatives of the alcohols or phenols mentioned are, in particular, the corresponding metal alcoholates or phenolates, preferably of an alkali metal, such as sodium or potassium.

The esterification is advantageously carried out in the presence of an inert solvent. Particularly suitable solvents are ethers, such as diethyl ether, di-n-butyl ether, THF, dioxane or anisole, ketones, such as acetone, butanone or cyclohexanone, amides, such as dimethylformamide or phosphoric acid hexamethyltriamide, hydrocarbons, such as benzene, toluene or xylene, halogenohydrocarbons, such as carbon tetrachloride, dichlormethane or tetrachloroethylene, and sulfoxides, such as dimethylsulfoxide or sulfolane. Water-immiscible solvents can simultaneously be advantageously used for azeotropic distillation of the water formed during the esterification. An excess of an organic base, for example pyridine, quinoline or triethylamine, can occasionally also be used as the solvent for the esterification. An additional, catalytic amount of 4-(N,N-dimethylamino)-pyridine can accelerate the esterification. The esterification can also be carried out in the absence of a solvent, for example by heating the components in the presence of sodium acetate. The reaction temperature is usually between -50° and +250°, preferable between -20° and +80°. At these temperatures, the esterification reactions have as a rule ended after 15 minutes to 48 hours.

In detail, the reaction conditions for the esterification depend largely on the nature of the starting substances used. Thus, a free carboxylic acid is as a rule reacted with a free alcohol or phenol in the presence of a strong acid, for example a mineral acid, such as hydrochloric acid or sulfuric acid. A preferred reaction procedure is the reaction of an acid anhydride or, in particular, an acid chloride with an alcohol, preferably in a basic medium, bases which are of importance being, in particular, alkali metal

hydroxides, such as sodium hydroxide or potassium hydroxide, alkali metal carbonates or bicarbonates, such as sodium carbonate, sodium bicarbonate, potassium carbonate or potassium bicarbonate, alkali metal acetates, such as sodium acetate or potassium acetate, alkaline earth metal hydroxides, such as calcium hydroxide, or organic bases, such as triethylamine, pyridine, lutidine, collidine or quinoline. Another preferred embodiment of the esterification comprises first converting the alcohol or phenol into the sodium alcoholate or phenolate or potassium alcoholate or phenolate, for example by treatment with ethanolic sodium hydroxide solution or potassium hydroxide solution, isolating this product and suspending it in acetone or diethyl ether, together with sodium bicarbonate or potassium carbonate, with stirring, and adding a solution of the acid chloride or anhydride in diethyl ether, acetone or diemthylformamide to this suspension, advantageously at temperatures between about -25° and +20°.

Alkoxy compounds of the formula III (o = O) can be obtained by alkylation of the corresponding phenols of the formula VIIIe, the phenol preferably first being converted into a phenolate for example into the alkali metal phenolate by treatment with NaOH, KOH, $Na_2CO_3$ or $K_2CO_3$. This phenolate can then be reacted with the corresponding reactive derivative of the alcohols of the formula VIIb in an inert solvent such as acetone, DMF or dimethylsulfoxide or an excess of aqueous or aqueous alcoholic NaOH or KOH at temperatures between 0° and 100 °C. Reactive derivatives of the alcohols of the formula VIIb are preferably the sulfonates as for example toluene sulfonate or mesilated.

The alcohol of the formula VIIb

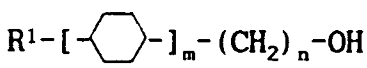

VIIb

and the corresponding phenol can also be treated with triphenyl phosphine and diethyl azodicarboxylate as described by O. Mitsunobu, Synthesis 1981, 1.

The 3-cyclohexylpropanols (n = 3) of the formula VIIb can be obtained by reduction of VIIa (n = 2) with, for example, lithium alanate.

The compounds of the formula IV can be obtained according to the following schemes:

**Scheme 1**

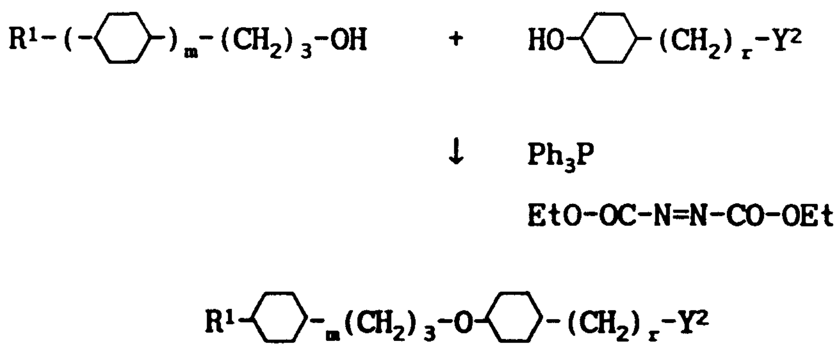


**Scheme 2**

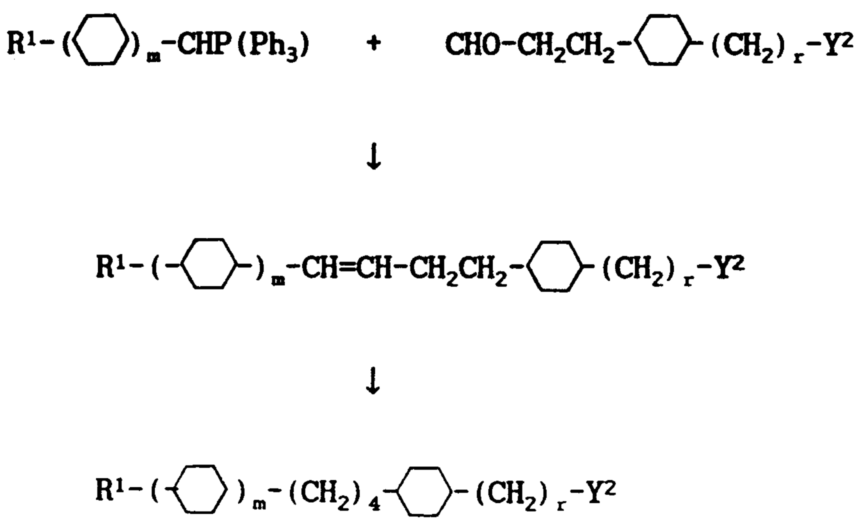

The compounds of the formulae I, II, III, V and VI wherein the bridging group is an allylic ether of the formula $-CH=CH-CH_2-O-$ can be obtained according to scheme 3

**Scheme 3**

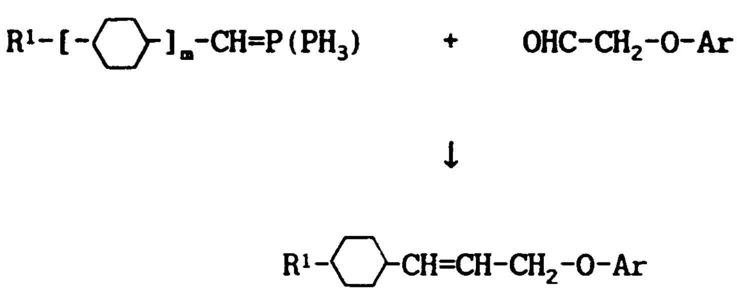

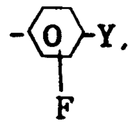, 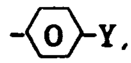, 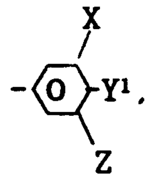, 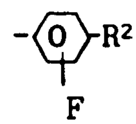 and

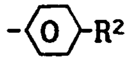

The compounds of the formulae I, III and V wherein the bridging groups is an acrylic acid ester can be obtained according to scheme 4.

**Scheme 4**

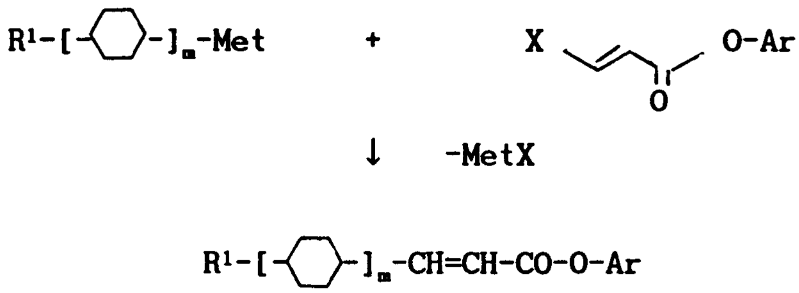


Met: Li, Mg, Br, ZnBr

X : Cl, Br, J

The compounds of the formula III, wherein $E^2$ is $(CH_2)_3$, o is O and $Y^1$ is $OCF_2H$, can be obtained according to scheme 5

**Scheme 5**

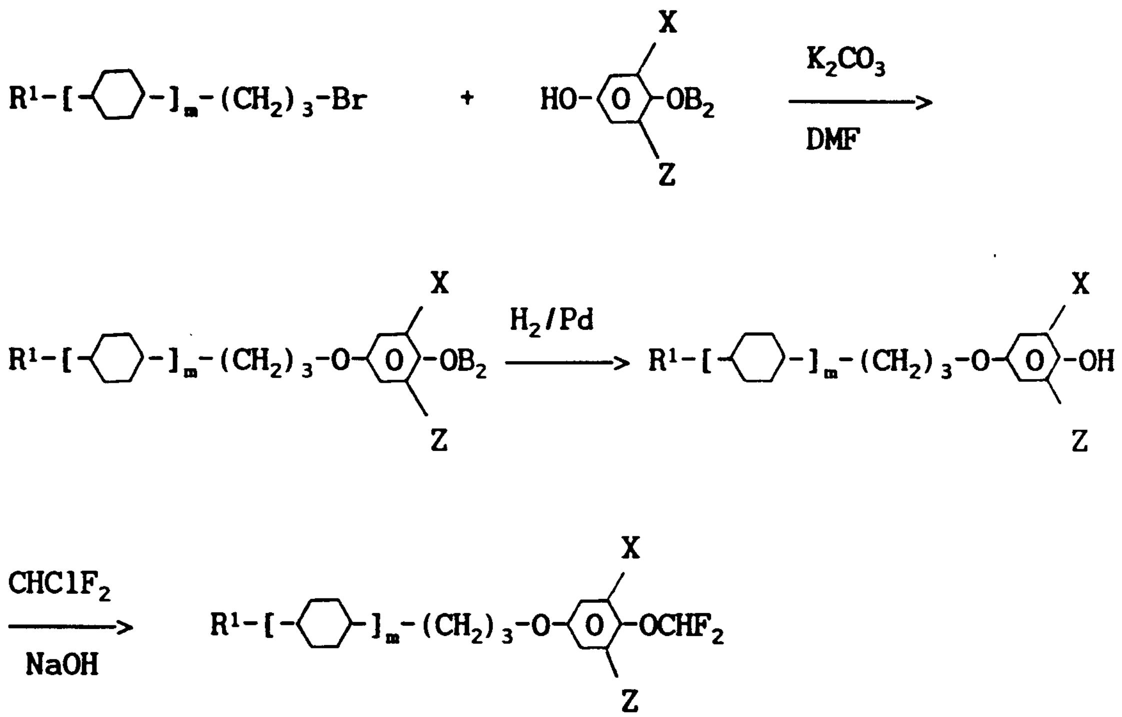


The compounds of the formula III, wherein $E_2$ is $(CH_2)_3$, o is O and $Y_1$ is $CF_3$ can be obtained according to scheme 6

**Scheme 6**

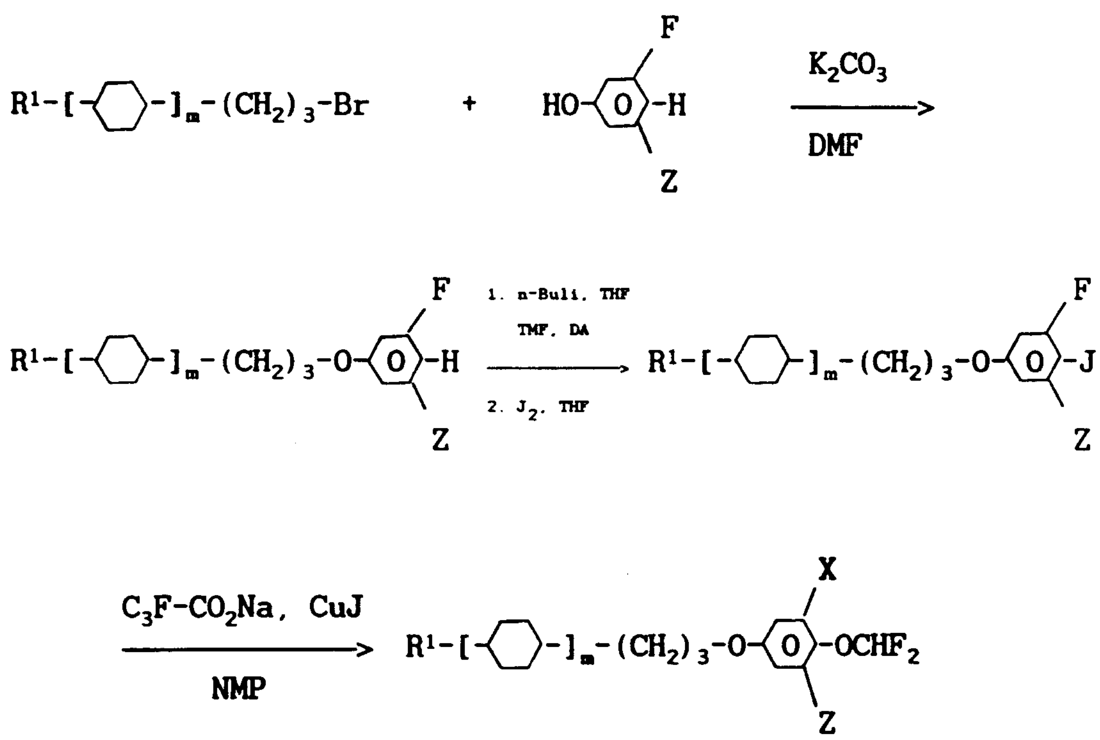

In addition to one or more compounds of formulae I to VI the liquid crystal compositions according to the invention preferably contain 2-40 components and in particular 4-30 components. Liquid crystal compositions being composed of one or more compounds of formulae I, II, III, IV, V and VI and 7-25 other components are especially preferred.

These additional components are preferably chosen from the nematic or nematogenic (monotropic or isotropic) substances; in particular from the classes of azoxybenzenes, benzylideneanilines, biphenyls, terphenyls, phenyl or cyclohexyl benzoates, phenyl or cyclohexyl cyclohexanecarboxylates, phenyl or cyclohexyl cyclohexylbenzoates, phenyl or cyclohexyl cyclohexylcyclohexanecarboxylates, cyclohexylphenylbenzoates, cyclohexylphenyl cyclohexanecarboxylates, cyclohexylphenyl cyclohexylcyclohexanecarboxylates, phenylcyclohexanes, cyclohexylbiphenyls, phenylcyclohexylcyclohexanes, cyclohexylcyclohexanes, cyclohexylcyclohexenes, cyclohexylcyclohexylcyclohexene, 1,4-bis-cyclohexylbenzenes, 4,4′-bis-cyclohexylbiphenyls, phenyl- or cyclohexylpyrimidines, phenyl- or cyclohexylpyridines, phenyl- or cyclohexyldioxanes, phenyl- or cyclohexyl-1,3-dithianes, 1,2-diphenylethanes, 1,2-dicyclohexylethanes, 1-phenyl-2-cyclohexylethanes, 1-cyclohexyl-2-(4-phenyl-cyclohexyl)-ethanes, 1-cyclohexyl-2-biphenylethanes, 1-phenyl-2-cyclohexylphenylethanes, optionally halogenated stilbenes, benzyl phenyl ethers, tolanes and substituted cinnamic acids.

The 1,4-phenylene groups of these compounds may be fluorinated.

The most important compounds which are possible constituents of liquid crystal compositions according to the invention can be characterized by the formulae 1, 2, 3, 4 and 5:

R′-L-U-R″

R′-L-COO-U-R″

R′-L-OOC-U-R″

R′-L-$CH_2CH_2$-U-R″

R′-L-C≡C-U-R″

In the formulae 1, 2, 3, 4 and 5 L and U may be equal or different from each other. L and U independently from each other denote a bivalent residue selected from the group consisting of -Phe-, -Cyc-, -Phe-Phe-, -Phe-Cyc-, -Cyc-Cyc-, -Pyr-, -Dio-, -G-Phe-, -G-Cyc- and their mirror images; in this compilation of residues Phe denotes unsubstituted or fluorinated 1,4-phenylen, Cyc trans- 1,4-cyclohexylene or 1,4-cyclohexenylen, Pyr pyrimidine-2,5-diyl or pyridine-2,5-diyl, Dio 1,3-dioxane-2,5-diyl and G 2-(trans-1,4-cyclohexyl)-ethyl, pyrimidine-2,5-diyl, pyridine-2,5-diyl or 1,3-dioxane-2,5-diyl.

One of the residues L and U is preferably Cyc, Phe or Pyr. U preferably denotes Cyc, Phe or Phe-Cyc. The liquid crystal media according to the invention preferably contain one or more components selected from the compounds of formulae 1, 2, 3, 4 and 5 with L and U meaning Cyc, Phe and Pyr, said liquid crystal media further containing at the same time one or more components selected from the compounds of formulae 1, 2, 3, 4 and 5 with one of the residues L and U denoting Cyc, Phe and Pyr and the other residue being selected from the group consisting of -Phe-Phe-, -Phe-Cyc-, -Cyc-Cyc-, -G-Cyc-, said liquid crystal media containing in addition to this optionally one or more components selected from the compounds of formulae 1, 2, 3, 4 and 5 with L and U being selected from the group consisting of -Phe-Cyc-, -Cyc-Cyc-, -G-Phe- and -G-Cyc.

In a preferred subgroup of the compounds of formulae 1, 2, 3, 4 and 5 (subgroup 1) R′ and R″ are independently from each other alkyl, alkenyl, alkoxy, alkenoxy with up to 8 carbon atoms. R′ and R″ differ from one another in most of these compounds, one of the residues usually being alkyl or alkenyl. In another preferred subgroup of the compounds of formulae 1, 2, 3, 4 and 5 (subgroup 2) R″ denotes -CN, -CF3, -F, -Cl or -NCS while R′ has the meaning indicated in subgroup 1 and is preferably alkyl or alkenyl. Other variants of the envisaged substituents in the compounds of formulae 1, 2, 3, 4 and 5 are also customary. Many such substances are commercially available. All these substances are obtainable by methods which are known from the literature or by analogous methods.

The liquid crystal compositions according to the invention preferably contain in addition to components selected from subgroup 1 also components of subgroup 2, the percentage of these components being as follows:

subgroup 1: 20 to 90 %, in particular 30 to 90 %

subgroup 2: 10 to 50 %, in particular 10 to 50 %

In these liquid crystal media the percentages of the compounds according to the invention and the compounds of subgroup 1 and 2 may add up to give 100 %.

The compositions according to the invention preferably contain 1 to 40 %, in particular 5 to 30 % of the compounds according to the invention. Media containing more than 40 %, in particular 45 to 90 % of the compounds according to the invention are further preferred. The media contain preferably 3, 4 or 5 compounds according to the invention.

The compositions according to the invention are prepared in a manner which is customary per se. As a rule, the components are dissolved in one another, advantageously at elevated temperature. The liquid crystal compositions according to the invention can be modified by suitable additives so that they can be used in all the types of liquid crystal display devices.

Such additives are known to the expert and are described in detail in the literature (H. Kelker/R. Hatz, Handbook of Liquid Crystals, Verlag Chemie, Weinheim, 1980). For example, it is possible to add pleochroic dyestuffs to prepare colored guest-host systems or substances for modifying the dielectric anisotropy, the viscosity and/or the orientation of the nematic phases.

Preferably the compositions according to the invention are used in twisted nematic cells. Twisted nematic cells includes all types of displays, which are based on twisted nematic effect in particular the TN-LCD, the supertwisted STN- and OMI-LCDs, cells which are addressed by a thin film transistor (TFT-LCD) and the SBE-cells.

The following examples are to be construed as merely illustrative and not limitative. m.p. = melting point, c.p. = clearing point. In the foregoing and in the following all parts and percentages are by weight and the temperatures are set forth in degrees Celsius. "Customary work-up" means that water is added, the mixture is extracted with methylene chloride, the organic phase is seperated off, dried and evaporated, and the product is purified by crystallization and/or chromatography.

Further are:

C: crystalline-solid state, S: smectic phase (the index denoting the typ of smectic phase), N: nematic phase, Ch: cholesteric phase, I: isotropic phase. The number being embraced by 2 of these symbols denotes the temperature of phase change.

Example 1

Step 1: Preparation of 3-(trans-4-propylcyclohexyl)-propionic acid

Trans 4-propyl cyclohexyl methane tosylate (100 g) is dissolved in dry methylated spirits (500 ml) and slowly added to a suspension of sodio diethyl malonate prepared from sodium (7.42 g) and diethyl malonate (54.2 g) in dry ethanol (210 ml). The mixture is heated and stirred for 30 hours. After distilling off the ethanol, the mixture is extracted into ether (3 x 200 ml), washed with water and dried. After evaporation of the solvent the diester product solidifies. Yield 59 g Mpt 47 °C.

The diester (59 g) is refluxed for 2 hours with KOH (59 g), water (60 ml) and ethanol (200 ml), about 160 ml of ethanol is distilled off and water (60 ml) added. When cool, conc. H2SO4 (51 m) in water (130 ml) is added and the mixture is heated for 2 hours. After cooling the mixture is extracted with ether which after the usual workup gave the desired acid.

Step 2: Ester Synthesis

A mixture of the acid from Step 1 (0.023 m), 4-cyano-3-fluorophenol (0.034 m) and dicyclohexylcarbodiimide (0.025 m) in dichloromethane (180 ml) is stirred for 16 hours. The mixture is filtered, evaporated to dryness, chromatographed and crystallized to yield (4-cyano-3-fluorophenyl) 3-(trans-4-propylcyclohexyl)-propionate K 46.5 N (-8.7) I.

Analogously are obtained:

(4-cyano-3-fluorophenyl) 3-(trans-4-ethylcyclohexyl)-propionate
(4-cyano-3-fluorophenyl) 3-(trans-4-butylcyclohexyl)-propionate
(4-cyano-3-fluorophenyl) 3-(trans-4-pentylcyclohexyl)-propionate
(4-cyano-3-fluorophenyl) 3-(trans-4-hexylcyclohexyl)-propionate
(4-cyano-3-fluorophenyl) 3-(trans-4-heptylcyclohexyl)-propionate
(4-cyano-3-fluorophenyl) 3-(trans-4-octylcyclohexyl)-propionate
(4-cyano-3-fluorophenyl) 3-(trans,trans-4′-ethylbicyclohexyl-4-yl)-propionate
(4-cyano-3-fluorophenyl) 3-(trans,trans-4′-propylbicyclohexyl-4-yl)-propionate
(4-cyano-3-fluorophenyl) 3-(trans,trans-4′-butylbicyclohexyl-4-yl)-propionate
(4-cyano-3-fluorophenyl) 3-(trans,trans-4′-pentylbicyclohexyl-4-yl)-propionate
(4-cyano-3-fluorophenyl) 3-(trans,trans-4′-hexylbicyclohexyl-4-yl)-propionate
(4-cyano-3-fluorophenyl) 3-(trans,trans-4′-heptylbicyclohexyl-4-yl)-propionate
(4-cyano-3-fluorophenyl) 3-(trans,trans-4′-octylbicyclohexyl-4-yl)-propionate
(3,4-difluorophenyl) 3-(trans-4-ethylcyclohexyl)-propionate
(3,4-difluorophenyl) 3-(trans-4-propylcyclohexyl)-propionate

(3,4-difluorophenyl) 3-(trans-4-butylcyclohexyl)-propionate
(3,4-difluorophenyl) 3-(trans-4-pentylcyclohexyl)-propionate
(3,4-difluorophenyl) 3-(trans-4-hexylcyclohexyl)-propionate
(3,4-difluorophenyl) 3-(trans-4-heptylcyclohexyl)-propionate
(3,4-difluorophenyl) 3-(trans-4-octylcyclohexyl)-propionate
(3,4-difluorophenyl) 3-(trans,trans-4′-ethylbicyclohexyl-4-yl)-propionate
(3,4-difluorophenyl) 3-(trans,trans-4′-propylbicyclohexyl-4-yl)-propionate
(3,4-difluorophenyl) 3-(trans,trans-4′-butylbicyclohexyl4-yl)-propionate
(3,4-difluorophenyl) 3-(trans,trans-4′-pentylbicyclohexyl4-yl)-propionate
(3,4-difluorophenyl) 3-(trans,trans-4′-hexylbicyclohexyl-4-yl)-propionate
(3,4-difluorophenyl) 3-(trans,trans-4′-heptylbicyclohexyl-4-yl)-propionate
(3,4-difluorophenyl) 3-(trans,trans-4′-octylbicyclohexyl-4-yl)-propionate
(4-chloro-3-fluorophenyl) 3-(trans-4-ethylcyclohexyl)-propionate K 43.6 N (-27) I
(4-chloro-3-fluorophenyl) 3-(trans-4-propylcyclohexyl)-propionate
(4-chloro-3-fluorophenyl) 3-(trans-4-butylcyclohexyl)-propionate
(4-chloro-3-fluorophenyl) 3-(trans-4-pentylcyclohexyl)-propionate
(4-chloro-3-fluorophenyl) 3-(trans-4-hexylcyclohexyl)-propionate
(4-chloro-3-fluorophenyl) 3-(trans-4-heptylcyclohexyl)-propionate
(4-chloro-3-fluorophenyl) 3-(trans-4-octylcyclohexyl)-propionate
(4-chloro-3-fluorophenyl) 3-(trans,trans-4′-ethylbicyclohexyl)-propionate
(4-chloro-3-fluorophenyl) 3-(trans,trans-4′-propylbicyclohexyl)-propionate
(4-chloro-3-fluorophenyl) 3-(trans,trans-4′-butylbicyclohexyl)-propionate
(4-chloro-3-fluorophenyl) 3-(trans,trans-4′-pentylbicyclohexyl)-propionate
(4-chloro-3-fluorophenyl) 3-(trans,trans-4′-hexylbicyclohexyl)-propionate
(4-chloro-3-fluorophenyl) 3-(trans,trans-4′-heptylbicyclohexyl)-propionate
(4-chloro-3-fluorophenyl) 3-(trans,trans-4′-octylbicyclohexyl)-propionate
(4-fluorophenyl) 3-(trans,trans-4′-ethylbicyclohexyl)-propionate
(4-fluorophenyl) 3-(trans,trans-4′-propylbicyclohexyl)-propionate
(4-fluorophenyl) 3-(trans,trans-4′-butylbicyclohexyl)-propionate
(4-fluorophenyl) 3-(trans,trans-4′-pentylbicyclohexyl)-propionate
(4-fluorophenyl) 3-(trans,trans-4′-hexylbicyclohexyl)-propionate
(4-fluorophenyl) 3-(trans,trans-4′-heptylbicyclohexyl)-propionate
(4-fluorophenyl) 3-(trans,trans-4′-octylbicyclohexyl)-propionate
(4-propyl-3-fluorophenyl) 3-(trans-4-ethylcyclohexyl)-propionate
(4-propyl-3-fluorophenyl) 3-(trans-4-propylcyclohexyl)-propionate
(4-propyl-3-fluorophenyl) 3-(trans-4-butylcyclohexyl)-propionate
(4-propyl-3-fluorophenyl) 3-(trans-4-pentylcycloheryl)-propionate
(4-propyl-3-fluorophenyl) 3-(trans-4-hexylcyclohexyl)-propionate
(4-propyl-3-fluorophenyl) 3-(trans-4-heptylcyclohexyl)-propionate
(4-propyl-3-fluorophenyl) 3-(trans-4-octylcyclohexyl)-propionate
(4-propyl-3-fluorophenyl) 3-(trans,trans-4′-ethylbicyclohexyl-4-yl)-propionate
(4-propyl-3-fluorophenyl) 3-(trans,trans-4′-propylbicyclohexyl-4-yl)-propionate
(4-propyl-3-fluorophenyl) 3-(trans,trans-4′-butylbicyclohexyl-4-yl)-propionate
(4-propyl-3-fluorophenyl) 3-(trans,trans-4′-pentylbicyclohexyl-4-yl)-propionate
(4-propyl-3-fluorophenyl) 3-(trans,trans-4′-hexylbicyclohexyl-4-yl)-propionate
(4-propyl-3-fluorophenyl) 3-(trans,trans-4′-heptylbicyclohexyl-4-yl)-propionate
(4-propyl-3-fluorophenyl) 3-(trans,trans-4′-octylbicyclohexyl-4-yl)-propionate
(4-pentyl-3-fluorophenyl) 3-(trans,4-propylcyclohexyl)-propionate
(4-heptyl-3-fluorophenyl) 3-(trans,4-propylcyclohexyl)-propionate
(4-octyl-3-fluorophenyl) 3-(trans,4-propylcyclohexyl)-propionate
(4-pentyl-3-fluorophenyl) 3-(trans,4-pentylcyclohexyl)-propionate
(4-heptyl-3-fluorophenyl) 3-(trans,4-pentylcyclohexyl)-propionate
(4-octyl-3-fluorophenyl) 3-(trans,4-pentylcyclohexyl)-propionate
(4-pentyl-3-fluorophenyl) 3-(trans,4-heptylcyclohexyl)-propionate
(4-heptyl-3-fluorophenyl) 3-(trans,4-heptylcyclohexyl)-propionate
(4-octyl-3-fluorophenyl) 3-(trans,4-heptylcyclohexyl)-propionate
(4-pentyl-3-fluorophenyl) 3-(trans,4-octylcyclohexyl)-propionate
(4-heptyl-3-fluorophenyl) 3-(trans,4-octylcyclohexyl)-propionate

(4-octyl-3-fluorophenyl) 3-(trans,4-octylcyclohexyl)-propionate
(4-propyloxy-3-fluorophenyl) 3-(trans,4-pentylcyclohexyl)-propionate
(4-pentyloxy-3-fluorophenyl) 3-(trans,4-pentylcyclohexyl)-propionate
(4-heptyloxy-3-fluorophenyl) 3-(trans,4-propylcyclohexyl)-propionate
(4-octyloxy-3-fluorophenyl) 3-(trans,4-propylcyclohexyl)-propionate
(4-pentyl-3-fluorophenyl) 3-(trans,trans-4′-propylbicyclohexyl)-propionate
(4-heptyl-3-fluorophenyl) 3-(trans,trans-4′-propylbicyclohexyl) -propionate
(4-octyl-3-fluorophenyl) 3-(trans,trans-4′-propylbicyclohexyl)-propionate
(4-pentyl-3-fluorophenyl) 3-(trans,trans-4′-pentylbicyclohexyl)-propionate
(4-heptyl-3-fluorophenyl) 3-(trans,trans-4′-pentylbicyclohexyl)-propionate
(4-octyl-3-fluorophenyl) 3-(trans,trans-4′-pentylbicyclohexyl)-propionate
(4-pentyl-3-fluorophenyl) 3-(trans,trans-4′-heptylbicyclohexyl)-propionate
(4-heptyl-3-fluorophenyl) 3-(trans,trans-4′-heptylbicyclohexyl)-propionate
(4-octyl-3-fluorophenyl) 3-(trans,trans-4′-heptylbicyclohexyl)-propionate
(4-propyloxy-3-fluorophenyl) 3-(trans,trans-4′-pentylbicyclohexyl)-propionate
(4-pentyloxy-3-fluorophenyl) 3-(trans,trans-4′-pentylbicyclohexyl)-propionate
(4-heptyloxy-3-fluorophenyl) 3-(trans,trans-4′-pentylbicyclohexyl)-propionate

Example 2

Step 1: Preparation of 3-(trans-5-propylcyclohexyl)-1-propanole

The methyl ester (0.2 m) of the acid of Example 1 Step 1 is dissolved in dry ether (150 ml) and slowly added to a stirred and cooled mixture of $LiAlH_4$ (0.12 m) in dry ether (100 ml). After complete addition to mixture is stirred for 16 hours at 20 °C and then heated to reflux for 1 hour. After cooling, ethyl acetate followed by water and dilute $H_2SO_4$ are added. The organic layer is separated and the product isolated. Vacuum distillation affords the pure product.

Step 2: Synthesis of the Tosylate

A mixture of alcohol of Step 1 (0.1 m) and toluenesulphonyl chloride (0.1 m) is dissolved in pyridine and dichloromethane and the mixture stirred for 16 hours. The mixture is then repeatedly extracted with cold dilute hydrochloric acid. After drying and evaporation the tosylate is isolated.

Step 3: Etherification

A mixture of tosylate of step 2 (0.1 m), p-pentyl phenol (0.1 m), potassium carbonate (0.2 m) and butanone (200 ml) is stirred and refluxed for 12 hours. Solid is filtered off, butanone evaporated and the product purified by chromatography/crystallisation to yield 1-(trans-4-propylcyclohexyl)-3-(p-pentylphenyloxy)-propane.

Analogously are obtained
1-(trans-4-ethylcyclohexyl)-3-(p-pentylphenyloxy)-propane
1-(trans-4-butylcyclohexyl)-3-(p-pentylphenyloxy)-propane
1-(trans-4-pentylcyclohexyl)-3-(p-pentylphenyloxy)-propane
1-(trans-4-hexylcyclohexyl)-3-(p-pentylphenyloxy)-propane
1-(trans-4-heptylcyclohexyl)-3-(p-pentylphenyloxy)-propane
1-(trans-4-octylcyclohexyl)-3-(p-pentylphenyloxy)-propane
1-(trans-4-nonylcyclohexyl)-3-(p-pentylphenyloxy)-propane
1-(trans-4-ethylcyclohexyl)-3-(p-heptylphenyloxy)-propane
1-(trans-4-propylcyclohexyl)-3-(p-heptylphenyloxy)-propane
1-(trans-4-pentylcyclohexyl)-3-(p-heptylphenyloxy)-propane
1-(trans-4-hexylcyclohexyl)-3-(p-heptylphenyloxy)-propane
1-(trans-4-heptylcyclohexyl)-3-(p-heptylphenyloxy)-propane
1-(trans-4-octylcyclohexyl)-3-(p-heptylphenyloxy)-propane
1-(trans-4-ethylcyclohexyl)-3-(p-pentyloxyphenyloxy)-propane
1-(trans-4-propylcyclohexyl)-3-(p-pentyloxyphenyloxy)-propane
1-(trans-4-butylcyclohexyl)-3-(p-pentyloxyphenyloxy)-propane
1-(trans-4-pentylcyclohexyl)-3-(p-pentyloxyphenyloxy)-propane K 42 $S_A$ 48 N 53 I

1-(trans-4-hexylcyclohexyl)-3-(p-pentyloxyphenyloxy)-propane
1-(trans-4-heptylcyclohexyl)-3-(p-pentyloxyphenyloxy)-propane
1-(trans-4-octylcyclohexyl)-3-(p-pentyloxyphenyloxy)-propane
1-(trans-4-pentylcyclohexyl)-3-(3-fluoro-4-pentylphenyloxy)-propane
1-(trans-4-pentylcyclohexyl)-3-(3-fluoro-4-heptylphenyloxy)-propane
1-(trans-4-pentylcyclohexyl)-3-(3-fluoro-4-octylphenyloxy)-propane
1-(trans-4-pentylcyclohexyl)-3-(3-fluoro-4-pentyloxyphenyloxy)-propane
1-(trans-4-pentylcyclohexyl)-3-(3-fluoro-4-heptyloxyphenyloxy)-propane
1-(trans-4-pentylcyclohexyl)-3-(3-fluoro-4-octyloxyphenyloxy)-propane
1-(trans-4-pentylcyclohexyl)-3-(3-fluoro-4-cyanophenyloxy)-propane K 59 N (29) I
1-(trans-4-pentylcyclohexyl)-3-(3-fluoro-4-chlorophenyloxy)-propane
1-(trans-4-pentylcyclohexyl)-3-(3,4-difluorophenyloxy)-propane K 4 I
1-(trans,trans-4′-pentylbicyclohexyl-4-yl)-3-(p-pentylphenyl-oxy)-propane
1-(trans,trans-4′-pentylbicyclohexyl-4-yl)-3-(p-heptylphenyloxy)-propane
1-(trans,trans-4′-pentylbicyclohexyl-4-yl)-3-(p-pentyloxyphenyloxy)-propane
1-(trans,trans-4′-pentylbicyclohexyl-4-yl)-3-(3-fluoro-4-pentylphenyloxy)-propane
1-(trans,trans-4′-pentylbicyclohexyl-4-yl)-3-(3-fluoro-4-cyanophenyloxy)-propane
1-(trans,trans-4′-pentylbicyclohexyl-4-yl)-3-(3-fluoro-4-chlorophenyloxy)-propane
1-(trans,trans-4′-pentylbicyclohexyl-4-yl)-3-(3,4-difluorophenyloxy)-propane

Example 3

A mixture of 0.1 mol trans-4-n-pentylcyclohexylmethyl toluene sulfonate (obtainable by reduction of trans-4-n-pentylcyclohexane carboxylic acid with lithium alonate and esterification with toluene sulfonyl chloride), 0.1 mol trifluoromethoxy phenol, 0.1 mol potassium carbonate and 100 ml of dimethylformamide is heated to 100 °C for 15 hours. After destilling off the major part of the solvent water is added to the residue. After the aqueous layer is extracted with dichloromethane the organic layer is washed with water, dried with magnesium sulfate and the solvent is evaporated. 0.083 mol of 1-(trans-4-n-pentylcycloheryl)-methoxy-4-trifluormethoxy benzene are obtained.

Analogously are obtained:
1-(trans-4-ethylcyclohexyl)-methoxy-4-trifluomethoxybenzene
1-(trans-4-n-propylcyclohexyl)-methoxy-4-trifluomethoxybenzene
1-(trans-4-n-butylcyclohexyl)-methoxy-4-trifluomethoxybenzene
1-(trans-4-n-hexylcyclohexyl)-methoxy-4-trifluomethoxybenzene
1-(trans-4-n-heptylcyclohexyl)-methoxy-4-trifluomethoxybenzene
1-(trans-4-n-octylcyclohexyl)-methoxy-4-trifluomethoxybenzene
1-(trans,trans-4′-ethylbicyclohexyl-4-yl)-methoxy-4-trifluoromethoxybenzene
1-(trans,trans-4′-n-propylbicyclohexyl-4-yl)-methoxy-4-trifluoromethoxybenzene
1-(trans,trans-4′-n-butylbicyclohexyl-4-yl)-methoxy-4-trifluoromethoxybenzene
1-(trans,trans-4′-n-pentylbicyclohexyl-4-yl)-methoxy-4-trifluoromethoxybenzene
1-(trans,trans-4′-n-hexylbicyclohexyl-4-yl)-methoxy-4-trifluoromethoxybenzene
1-(trans,trans-4′-n-heptylbicyclohexyl-4-yl)-methoxy-4-trifluoromethoxybenzene
1-(trans,trans-4′-n-octylbicyclohexyl-4-yl)-methoxy-4-trifluoromethoxybenzene
1-(trans-4-ethylcyclohexyl)-methoxy-3-fluoro-4-trifluoromethoxybenzene
1-(trans-4-n-propylcyclohexyl)-methoxy-3-fluoro-4-trifluoromethoxybenzene
1-(trans-4-n-pentylcyclohexyl)methoxy-3-fluoro-4-trifluoromethoxybenzene
1-(trans-4-n-pentylcyclohexyl)-methoxy-3-fluoro-4-trifluoromethoxybenzene
1-(trans-4-n-hexylcyclohexyl)-methoxy-3-fluoro-4-trifluoromethoxybenzene
1-(trans-4-n-heptylcyclohexyl)-methoxy-3-fluoro-4-trifluoromethoxybenzene
1-(trans-4-n-octylcyclohexyl)-methoxy-3-fluoro-4-trifluoromethoxybenzene
1-(trans,trans-4′-ethylbicyclohexyl-4-yl)-methoxy-3-fluoro-4-trifluoromethoxybenzene
1-(trans,trans-4′-n-propylbicyclohexyl-4-yl)-methoxy-3-fluoro-4-trifluoromethoxybenzene
1-(trans,trans-4′-n-butylbicyclohexyl-4-yl)-methoxy-3-fluoro-4-trifluoromethoxybenzene
1-(trans,trans-4′-n-pentylbicyclohexyl-4-yl)-methoxy-3-fluoro-4-trifluoromethoxybenzene
1-(trans,trans-4′-n-hexylbicyclohexyl-4-yl)-methoxy-3-fluoro-4-trifluoromethoxybenzene
1-(trans,trans-4′-n-heptylbicyclohexyl-4-yl)methoxy-3-fluoro-4-trifluoromethoxybenzene
1-(trans,trans-4′-n-octylbicyclohexyl-4-yl)-methoxy-3-fluoro-4-trifluoromethoxybenzene

Example 4

A stream of ammonia is bubbled through a solution of 4-[trans-4-(trans-4-n-pentylcyclohexyl)-cyclohexyl-]methoxyanilin (0.01 mol, obtained by reaction of [trans-4-(trans-4-(trans-4-n-pentyl-cyclohexyl)-cyclohexyl]-methyl-toluenesulfonate with 4-acetamidophenol in the presence of potassium carbonate and dimethylformamide followed by the saponification of the acetanilide) for 10 minutes . 0.8 g of $CS_2$ are added and the ammonia is bubbled through until saturation. A solution of phosgene in toluene (6 ml, 20 %) is added and stirred for 30 minutes. After filtration evaporation and crystallization 4-[trans-4-(4-n-pentyl-cyclohexyl)-cyclohexyl]-methoxyphenylisothiocyanate are obtained.

Analogously are obtained:

4-[trans-4-(trans-4-ethylcyclohexyl)-cyclohexyl]-methoxyphenylisothiocyanate
4-[trans-4-(trans-4-n-propylcyclohexyl)-cyclohexyl]-methoxyphenylisothiocyanate
4-[trans-4-(trans-4-n-butylcyclohexyl)-cyclohexyl]-methoxyphenylisothiocyanate
4-[trans-4-(trans-4-n-hexylcyclohexyl)-cyclohexyl]-methoxyphenylisothiocyanate
4-[trans-4-(trans-4-n-heptylcyclohexyl)-cyclohexyl]-methoxyphenylisothiocyanate
4-[trans-4-(trans-4-n-octylcyclohexyl)-cyclohexyl]-methoxyphenylisothiocyanate
4-[trans-4-(trans-4-ethylcyclohexyl)-cyclohexyl]-methoxy-2-fluorophenylisothiocyanate
4-[trans-4-(trans-4-n-propylcyclohexyl)-cyclohexyl]-methoxy-2-fluorophenylisothiocyanate
4-[trans-4-(trans-4-n-butylcyclohexyl)-cyclohexyl]-methoxy-2-fluorophenylisothiocyanate
4-[trans-4-(trans-4-n-pentylcyclohexyl)-cyclohexyl]-methoxy-2-fluorophenylisothiocyanate
4-[trans-4-(trans-4-n-hexylcyclohexyl)-cyclohexyl]-methoxy-2-fluorophenylisothiocyanate
4-[trans-4-(trans-4-n-heptylcyclohexyl)-cyclohexyl]-methoxy-2-fluorophenylisothiocyanate
4-[trans-4-(trans-4-n-octylcyclohexyl)-cyclohexyl]-methoxy-2-fluorophenylisothiocyanate
4-trans-4-trans-4-ethylcyclohexyl)-cyclohexyl-methoxy-2,6-difluorophenylisothiocyanate
4-trans-4-trans-4-n-propylcyclohexyl)-cyclohexyl-methoxy-2,6-difluorophenylisothiocyanate
4-trans-4-trans-4-n-butylcyclohexyl)-cyclohexyl-methoxy-2,6-difluorophenylisothiocyanate
4-trans-4-trans-4-n-pentylcyclohexyl)-cyclohexyl-methoxy-2,6-difluorophenylisothiocyanate
4-trans-4-trans-4-n-hexylcyclohexyl)-cyclohexyl-methoxy-2,6-difluorophenylisothiocyanate
4-trans-4-trans-4-n-heptylcyclohexyl)-cyclohexyl-methoxy-2,6-difluorophenylisothiocyanate
4-trans-4-trans-4-n-octylcyclohexyl)-cyclohexyl-methoxy-2,6-difluorophenylisothiocyanate

Example 4

A solution of dicyclohexylcarbodiimide (0.01 mol) in dichloromethane (15 ml) is added to a mixture of 3-[trans-4-(trans-4-n-pentylcyclohexyl)-cyclohexyl]-propionic acid (0.001 mol, obtained from 4-trans-(trans-4-n-pentylcyclohexyl)-cyclohexane carboxylchloride via Rosemund-reduction to the corresponding aldehyde, Knoevenagel-condensation with malonic acid and hydrogenation of double bond), 4-dimethylaminopyridine (0,001 mol), 4-trifluoromethylphenol (0.01 mol) and dichloromethane (15 ml) at 10 °C under stirring. The mixture is stirred at room temperature for 15 hours. After filtration from silic gel the solvent is evaporated. (4-Trifluoromethylphenyl) 3-[trans-4-(trans-4-n-pentylcyclohexyl)-cyclohexyl]-propionate are obtained.

Analogously are obtained:

(4-trifluoromethylphenyl) 3-[trans-4-(trans-4-ethylcyclohexyl)-cyclohexyl]-propionate
(4-trifluoromethylphenyl) 3-[trans-4-(trans-4-n-propylcyclohexyl)-cyclohexyl]-propionate
(4-trifluoromethylphenyl) 3-[trans-4-(trans-4-n-butylcyclohexyl)-cyclohexyl]-propionate
(4-trifluoromethylphenyl) 3-[trans-4-(trans-4-n-hexylcyclohexyl)-cyclohexyl]-propionate
(4-trifluoromethylphenyl) 3-[trans-4-(trans-4-n-heptylcyclohexyl)-cyclohexyl]-propionate
(4-trifluoromethylphenyl) 3-[trans-4-(trans-4-n-octylcyclohexyl)-cyclohexyl]-propionate
(4-trifluoromethylphenyl) 3-(trans-4-ethylcyclohexyl)-propionate
(4-trifluoromethylphenyl) 3-(trans-4-n-propylcyclohexyl)-propionate
(4-trifluoromethylphenyl) 3-(trans-4-n-butylcyclohexyl)-propionate
(4-trifluoromethylphenyl) 3-(trans-4-n-pentylcyclohexyl)-propionate
(4-trifluoromethylphenyl) 3-(trans-4-n-hexylcyclohexyl)-propionate
(4-trifluoromethylphenyl) 3-(trans-4-n-heptylcyclohexyl)-propionate
(4-trifluoromethylphenyl) 3-(trans-4-n-octylcyclohexyl)-propionate
(4-trifluoromethoxyphenyl) 3-(trans-4-ethylcyclohexyl)-propionate
(4-trifluoromethoxyphenyl) 3-(trans-4-n-propylcyclohexyl)-propionate
(4-trifluoromethoxyphenyl) 3-(trans-4-n-butylcyclohexyl)-propionate
(4-trifluoromethoxyphenyl) 3-(trans-4-n-pentylcyclohexyl)-propionate

(4-trifluoromethoxyphenyl) 3-(trans-4-n-hexylcyclohexyl)-propionate
(4-trifluoromethoxyphenyl) 3-(trans-4-n-heptylcyclohexyl)-propionate
(4-trifluoromethoxyphenyl) 3-(trans-4-n-octylcyclohexyl)-propionate
(4-difluoromethoxyphenyl) 3-(trans-4-ethylcyclohexyl)-propionate
(4-difluoromethoxyphenyl) 3-(trans-4-n-propylcyclohexyl)-propionate
(4-difluoromethoxyphenyl) 3-(trans-4-n-butylcyclohexyl)-propionate
(4-difluoromethoxyphenyl) 3-(trans-4-n-pentylcyclohexyl)-propionate
(4-difluoromethoxyphenyl) 3-(trans-4-n-hexylcyclohexyl)-propionate
(4-difluoromethoxyphenyl) 3-(trans-4-n-heptylcyclohexyl)-propionate
(4-difluoromethoxyphenyl) 3-(trans-4-n-octylcyclohexyl)propionate
(3-fluoro-4-trifluoromethylphenyl) 3-(trans-4-ethylcyclohexyl)-propionate
(3-fluoro-4-trifluoromethylphenyl) 3-(trans-4-n-propylcyclohexyl)-propionate
(3-fluoro-4-trifluoromethylphenyl) 3-(trans-4-n-butylcyclohexyl)-propionate
(3-fluoro-4-trifluoromethylphenyl) 3-(trans-4-n-pentylcyclohexyl)-propionate
(3-fluoro-4-trifluoromethylphenyl) 3-(trans-4-n-hexylcyclohexyl)-propionate
(3-fluoro-4-trifluoromethylphenyl) 3-(trans-4-n-heptylcyclohexyl)-propionate
(3-fluoro-4-trifluoromethylphenyl) 3-(trans-4-n-octylcyclohexyl)-propionate
(3,4,5-trifluorphenyl) 3-(trans-4-ethylcyclohexyl)-propionate
(3,4,5-trifluorphenyl) 3-(trans-4-propylcyclohexyl)-propionate
(3,4,5-trifluorphenyl) 3-(trans-4-butylcyclohexyl)-propionate
(3,4,5-trifluorphenyl) 3-(trans-4-pentylcyclohexyl)-propionate
(3,4,5-trifluorphenyl) 3-(trans-4-hexylcyclohexyl)-propionate
(3,4,5-trifluorphenyl) 3-(trans-4-heptylcyclohexyl)-propionate
(3,4,5-trifluorphenyl) 3-(trans-4-octylcyclohexyl)-propionate
(3,5-difluoro-4-trifluoromethylphenyl) 3-(trans-4-ethylcyclohexyl)-propionate
(3,5-difluoro-4-trifluoromethylphenyl) 3-(trans-4-n-propylcyclohexyl)-propionate
(3,5-difluoro-4-trifluoromethylphenyl) 3-(trans-4-n-butylcyclohexyl)-propionate
(3,5-difluoro-4-trifluoromethylphenyl) 3-(trans-4-n-pentylcyclohexyl)-propionate
(3,5-difluoro-4-trifluoromethylphenyl) 3-(trans-4-n-hexylcyclohexyl)-propionate
(3,5-difluoro-4-trifluoromethylphenyl) 3-(trans-4-n-heptylcyclohexyl)-propionate
(3,5-difluoro-4-trifluoromethylphenyl) 3-(trans-4-n-octylcyclohexyl)-propionate
(3,4,5-trifluorophenyl) 3-(trans,trans-4′-ethylbicyclohexyl)-propionate
(3,4,5-trifluorophenyl) 3-(trans,trans-4′-n-propylbicyclohexyl)-propionate
(3,4,5-trifluorophenyl) 3-(trans,trans-4′-n-butylbicyclohexyl)-propionate
(3,4,5-trifluorophenyl) 3-(trans,trans-4′-n-pentylbicyclohexyl)-propionate
(3,4,5-trifluorophenyl) 3-(trans,trans-4′-n-octylbicyclohexyl)-propionate

Example 6

A mixture of 3-[trans-4-(trans-4-n-pentylcyclhexyl)-cyclohexyl]-propyl-toluenesulfonate (0.1 mol, obtained from the acid described in example 4 by reduction with lithium alanate and esterification with p-toluenesulfonylchloride), 4-fluorophenol (0.1 mol), potassium carbonate (0.1 mol) and dimethylformamide (100 ml) are heated to 100 °C for 15 hours.

After work-up as described in example 1 4-{3-[trans-4-(trans-4-n-pentylcyclohexyl)-cyclohexyl]-propyloxy}-fluorobenzene are obtained.

Analogously are obtained:

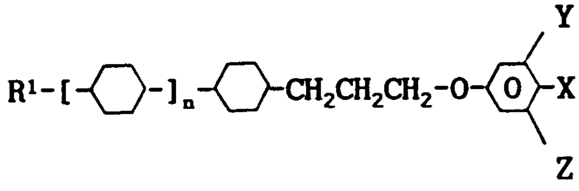

| $R^1$ | m | X | Y | Z |
|---|---|---|---|---|
| $C_3H_7$ | 1 | F | H | H |
| $C_7H_{15}$ | 1 | F | H | H |
| $C_3H_7$ | 0 | F | F | H |
| $C_5H_{11}$ | 0 | F | F | H |
| $C_7H_{15}$ | 0 | F | F | H |
| $C_3H_7$ | 0 | $CF_3$ | H | H |
| $C_3H_7$ | 0 | F | F | F |
| $C_3H_7$ | 0 | $CF_3$ | F | H |
| $C_3H_7$ | 0 | $OCF_3$ | F | H |
| $C_3H_7$ | 1 | F | F | F |
| $C_5H_{11}$ | 1 | F | F | F |
| $C_7H_{15}$ | 1 | F | F | F |
| $C_3H_7$ | 1 | $CF_3$ | H | H |
| $C_3H_7$ | 1 | $OCF_3$ | H | H |

Example 7

Preparation of 4-(trans,trans-4'-pentylbicyclohexyl-4-yl-methoxy)-2-fluoro-chlorobenzene trans-trans-4-pentylbicyclohexyl methyl iodide (0.02 m) is heated under reflux and stirred with butanone (200 ml) potassium carbonate (0.04 m) and 4-chloro 3-fluorophenol (0.021 m) for 16 hours. The mixture filtered and evaporated to dryness. The product is purified by column chromatography and crystallisation from ethanol and shows K 87 N 137.9 I.

Analogously are obtained

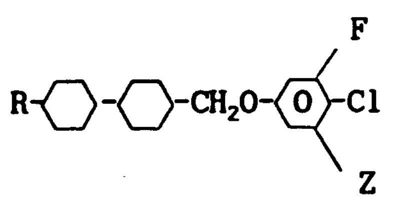

Example 8

A mixture of 3-(trans-4-propylcyclohexyl)-propyl-toluenesulfonate (0.1 mol, cp. Example 6), 3,5-difluorophenol (0.1 mol), potassium carbonate (0.1 mol) and dimethylformamide (10 ml) are heated to 100 °C for 15 hours. 62 ml of n-Buli (15 % in hexane) are added to a mixture of the obtained compound (90 mmol/l / cp. Scheme 6), TMEDA (100 mmol) and 100 ml THF at -70 °C. After 1 hour of stirring at -70 °C a solution of iodine (94 mmol) in 50 ml THF is added and 0.5 hrs stirred. After warning up to -30 °C 30 ml

water and 30 ml of a dilute solution of sodium hydrogen sulfite are added. After costumary work-up 4-[3-(trans-4-propylcyclohexyl)-propyloxy]-2,6-difluoro-iodobenzene are obtained.

A mixture of this iodobenzene derivative (76 mmol), potassium fluoride (152 mmol), sodium trifluoroacetate (336 mmol) and 1600 ml N-methylpyrolidone (NMP) are heated to 70 °C and 800 ml of NMP are distilled off at 4 mbar. CuI (152 mmol) is added to the reaction mixture and stirred for 5 hours at 160 °C. Then 800 ml NMP are distilled off. After the mixture is cooled to room temperature, 800 ml methyl-tert.-butylether are added. After customary work-up 4-[3-trans-4-propylcyclohexy)-propyloxy]-2,6-difluorotrifluoromethylbenzene are obtained.

Analogously are obtained:

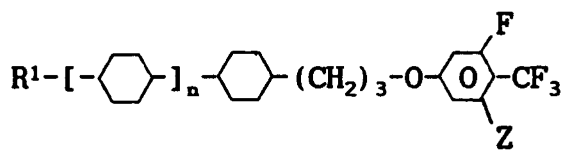

| $R^1$ | n | Z |
|---|---|---|
| $C_5H_{11}$ | 0 | F |
| $C_7H_{15}$ | 0 | F |
| $C_5H_7$ | 0 | H |
| $C_5H_{11}$ | 0 | H |
| $C_7H_{15}$ | 0 | H |
| $C_3H_7$ | 1 | H |
| $C_5H_{11}$ | 1 | H |
| $C_7H_{15}$ | 1 | H |
| $C_3H_7$ | 1 | F |
| $C_5H_{11}$ | 1 | F |
| $C_7H_{15}$ | 1 | F |

Example 9

A mixture of 4-[3-(trans-4-Pentylcyclohexyl)-propyloxy]-2-fluoro-phenol (10 mmol/obtained from 3-(trans-4-pentylcyclohexyl)-propyl-toluene sulfonate and 4-benzyloxy-3-fluorophenol as described in Example 6 followed by hydrogenolytic clearage of the benzylic ether), 50 ml THF, an aqueous solution of sodium hydroxide (32 %, 3.1 g) and tetrabutylammoniumhydrogen sulfate (0.5 g) is heated to 50 °C. Chlorodifluoromethane is added until it condenses on a condenser that is cooled by dry-ice.

After costumary work-up 4-[3-(trans-4-pentylcyclohexyl)-propyloxy]-2-fluorodifluoromethoxybenzene are obtained.

Analogously are obtained:

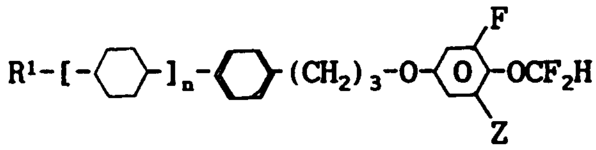

| R¹ | n | Z |
|---|---|---|
| $C_3H_7$ | 0 | H |
| $C_7H_{15}$ | 0 | H |
| $C_3H_7$ | 0 | F |
| $C_5H_{11}$ | 0 | F |
| $C_7H_{15}$ | 0 | F |
| $C_3H_7$ | 1 | H |
| $C_5H_{11}$ | 1 | H |
| $C_7H_{15}$ | 1 | H |
| $C_3H_7$ | 1 | F |
| $C_5H_{11}$ | 1 | F |
| $C_7H_{15}$ | 1 | F |

## Claims

**1.** A nematic liquid crystalline composition comprising a group (A) with one or more components exhibiting a positive dielectric anisotropy ($\Delta\epsilon$) and a group (B) with one or more components exhibiting a neutral dielectric anisotropy characterized in that

(a) at least one component of group (A) is a compound of the formula I

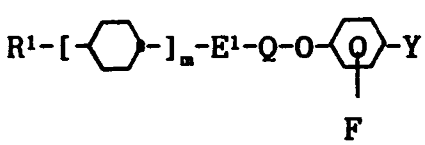

(I)

wherein

$R^1$ is an alkyl or alkenyl residue each with up to 16 C atoms, wherein one or two non-adjacent $CH_2$-groups of these residues may be replaced by -O-, -S-, -CO-O-, or -O-CO-,

$E^1$ is $-CH_2CH_2-$ or $-CH=CH-$
Q is -CO- or $-CH_2-$,
Y is halogen or CN, and
m is 1 or 2
or a compound of the formula II

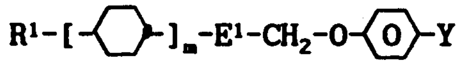

(II)

wherein $R^1$, $E^1$, Y and m have the meaning given for formula I,
or a compound of the formula III

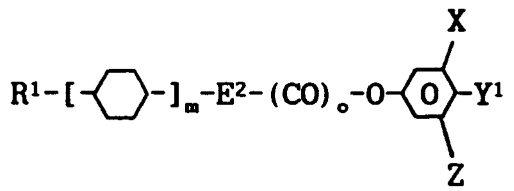

(III)

wherein
$R^1$ is an alkyl or alkenyl residue each with up to 16 C atoms, wherein one or two non-adjacent $CH_2$-groups of these residues may be replaced by -O-, -S-, -CO-O-, or -O-CO,
$E^2$ is $-(CH_2)_n-$ or $CH=CH-(CH_2)_{n-2}-$
X and Z are each independently H or F,
$Y^1$ is F, Cl, NCS, $CF_3$, $OCF_3$ or $OCF_2H$,
m is 1 or 2,
n is 1, 2 or 3,
o is 0 or 1
with the proviso that m is 2 or n is 2 or 3 in the case that $Y^1$ is F, Cl or NCS,
or a compound of the formula IV

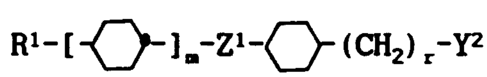

(IV)

wherein $R^1$ and m have the meaning given for formula I, and
$Z^1$ is $-(CH_2)_4-$, $-(CH_2)_3-O-$ or $-CH=CH-CH_2CH_2-$
$Y^2$ is $CF_3$, $OCF_3$ or $-CH=CH-CF_3$, and
r is 0 to 7,
or that
(b) at least one component of group (B) is a compound of the formula V

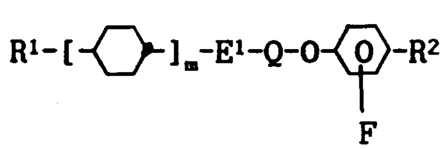

(V)

wherein $R^1$, $E^1$, Q and m have the meaning given for formula I, and

$R^2$ is an alkyl or alkenyl residue with up to 16 C atoms wherein one or two non-adjacent $CH_2$ groups of these residues may be replaced by -O-, -S-, -CO-O- or -O-CO-

or a compound of the formula VI

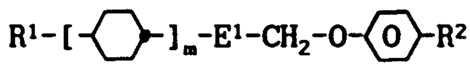

VI

wherein $R^1$, $R^2$, $E^1$ and m have the meaning given for formula V.

**2.** A nematic liquid crystalline composition according to claim 1, characterized in that

$E^1$ denotes $-CH_2CH_2-$

in the compounds of the formulae I, II, V and VI and

$E^2$ denotes $-(CH_2)_n-$,

in the compounds of the formula III.

**3.** A liquid crystalline composition according to claim 2, characterized in that at least one component of group (A) is selected from the compounds of the formulae Ia to In

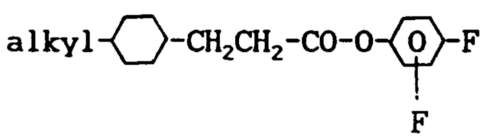

Ia

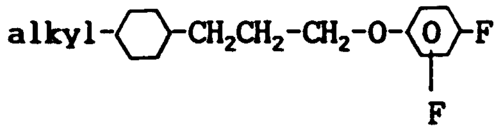 Ib

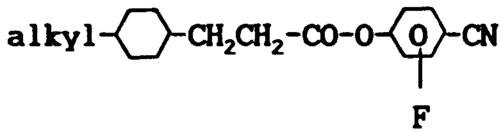 Ic

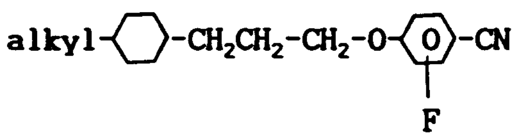 Id

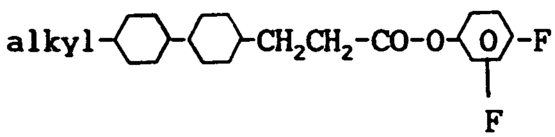 Ie

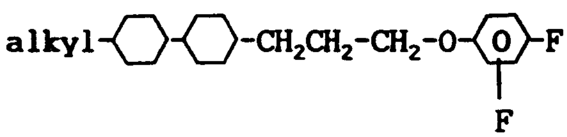 If

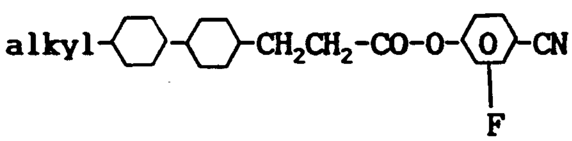 Ig

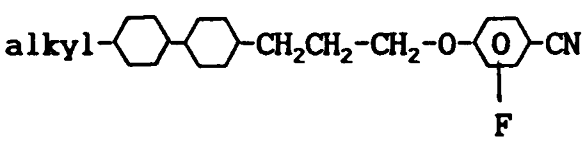 Ih

wherein alkyl denotes an alkyl residue with up to 16 C atoms.

**4.** A liquid crystalline composition according to claim 2 characterized in that at least one component of group (A) is selected from the compounds of the formulae IIa to IId:

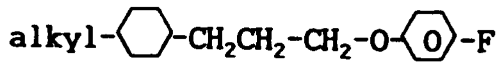 IIa

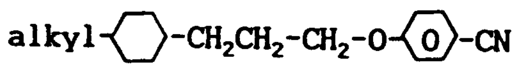 IIb

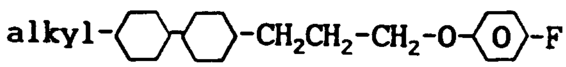 IIc

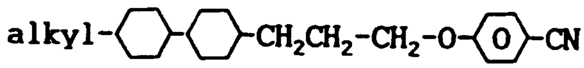 IId

5. A liquid crystalline composition according to claim 2 characterized in that at least one component of the group (B) is selected from the compounds of the formulae Va to Vd or VIa to VIb

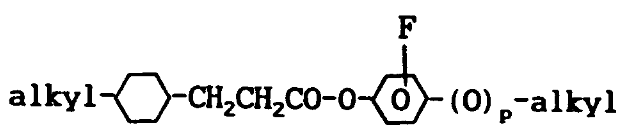 Va

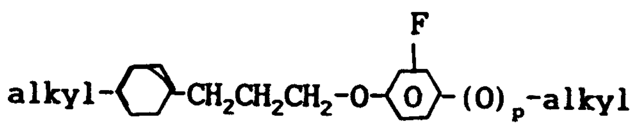 Vb

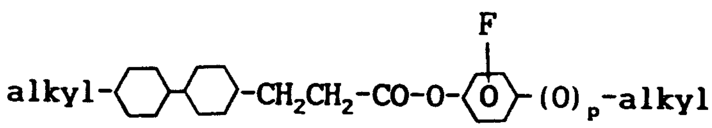 Vc

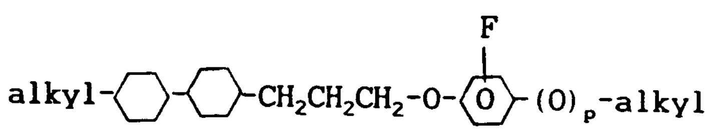 Vd

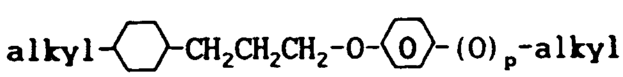 VIa

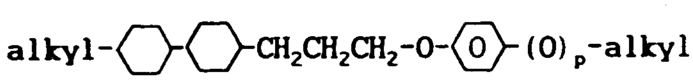 VIb

wherein p is 0 or 1.

6. A liquid crystalline composition according to at least one of the claims 1 to 5 characterized in that the dielectric anisotropy (Δε) of the group A is greater than +6.

7. A liquid crystal composition according to at least one of the claims 1 to 6 characterized in that the dielectric anisotropy ($\Delta\epsilon$) of the group B is -4 to +3.

8. A twisted nematic cell containing a liquid crystalline composition according to at least one of the claims 1 to 7.

9. A cell according to claim 8 characterized in that the twist angle is between 160 and 300°.

10. Cyclohexane derivatives of the formula III selected from the formula IIIa to IIII

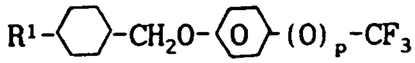

IIIa

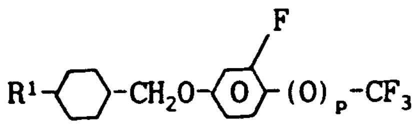

IIIb

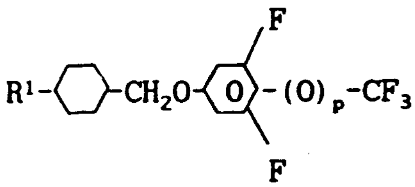

IIIc

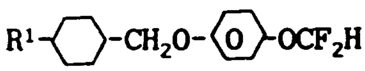

IIId

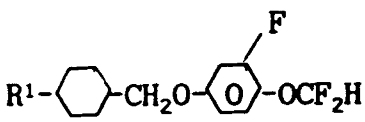

IIIe

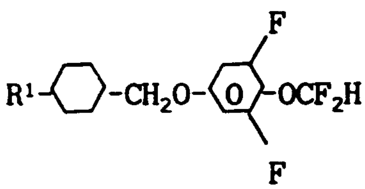

IIIf

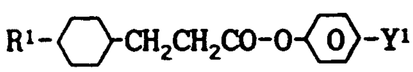

IIIg

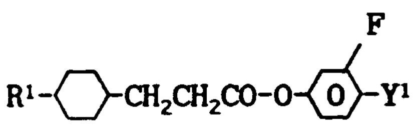

IIIh

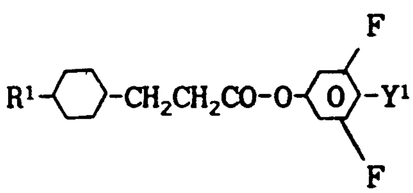

IIIi

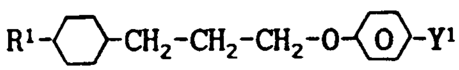

IIIj

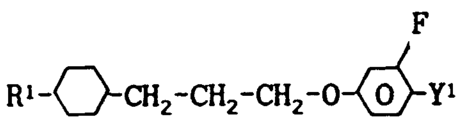

IIIk

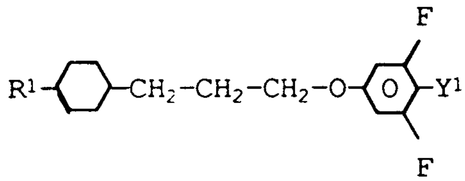

IIIl

wherein $R^1$ and $Y^1$ have the meaning given for formula III of claim 1, and p is 0 or 1, with the proviso that

a) the compounds of formula IIIj, wherein $Y^1$ is F, Cl or NCS, are excluded, and
b) the compounds of formula IIIk, wherein $Y^1$ is F are excluded.

**11.** Cyclohexane derivatives of the formula III selected from the formulae IIIm to IIIu

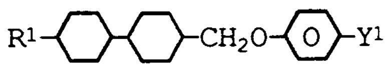

IIIm

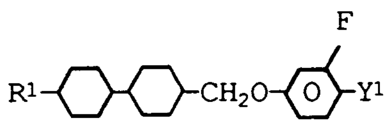

IIIn

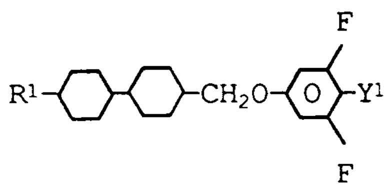

IIIo

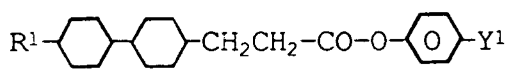

IIIp

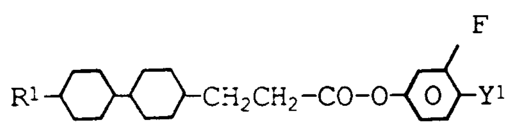

IIIq

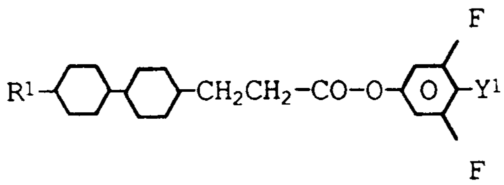

IIIr

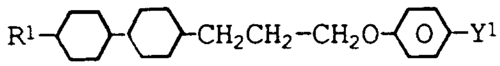

IIIs

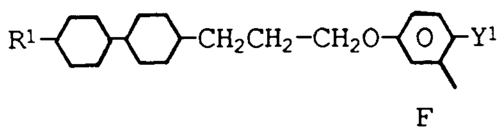

IIIt

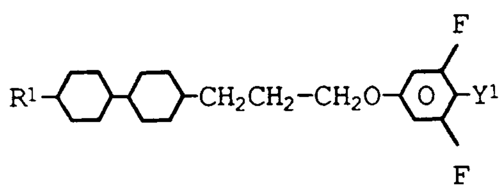

IIIu

wherein
$R^1$ and $Y^1$ have the meaning given for formula III of claim 1,
with the proviso that

c) the compounds of formula IIIs and IIIt, wherein $Y^1$ is F, are excluded.

**12.** A liquid crystalline medium according to claim 1 being a mixture of at least two compounds, characterized in that at least one compound is a cyclohexane derivative of the formula III

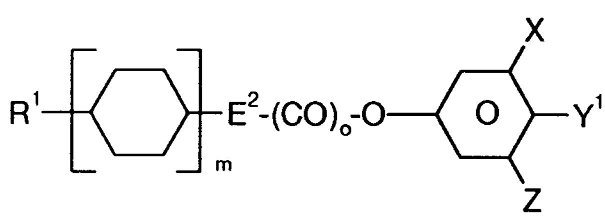

(III)

wherein $R^1$, $E^2$, X, Z, $Y^1$, m, n and o have the meaning given in claim 1.

**13.** A liquid crystalline medium according to claim 1 being a mixture of at least two components, characterized in that at least one component is a compound of the formula IV

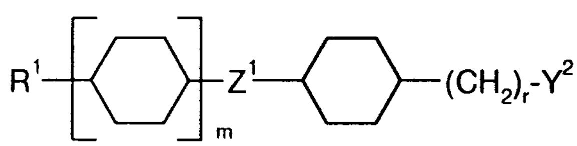

(IV)

wherein $R^1$, m, $Z^1$, $Y^2$ and r have the meaning given in claim 1.

## Patentansprüche

1. Nematische flüssigkristalline Zusammensetzung, bestehend aus einer Gruppe (A) mit einer oder mehreren Komponenten mit positiver dielektrischer Anisotropie ($\Delta\epsilon$) und einer Gruppe (B) mit einer oder mehreren Komponenten mit neutraler dielektrischer Anisotropie, dadurch gekennzeichnet, daß
   (a) mindestens eine Komponente aus Gruppe (A) eine Verbindung der Formel I

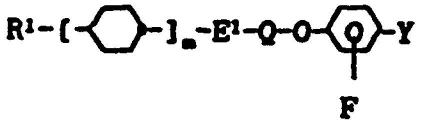

(I)

worin

| | |
|---|---|
| $R^1$ | einen Alkyl- oder Alkenylrest mit jeweils bis zu 16 C-Atomen, wobei eine oder zwei nicht benachbarte $CH_2$-Gruppen dieser Reste durch -O-, -S-, -CO-O- oder -O-CO- ersetzt sein können, |
| $E^1$ | $-CH_2CH_2-$ oder $-CH=CH-$ |
| Q | -CO- oder $-CH_2-$, |
| Y | Halogen oder CN und |
| m | 1 oder 2 bedeuten |

oder eine Verbindung der Formel II

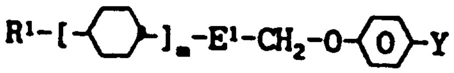

(II)

worin $R^1$, $E^1$, Y und m die für die Formel I angegebene Bedeutung besitzen,
oder eine Verbindung der Formel III

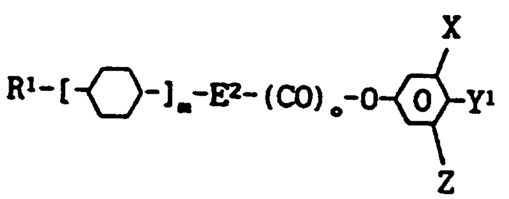

(III)

worin

| | |
|---|---|
| $R^1$ | einen Alkyl- oder Alkenylrest mit jeweils bis zu 16 C-Atomen, wobei eine oder zwei nicht benachbarte $CH_2$-Gruppen dieser Reste durch -O-, -S-, -CO-O- oder -O-CO-ersetzt sein können, |
| $E^2$ | $-(CH_2)_n-$ oder $-CH=CH-(CH_2)_{n-2}-$, |
| X und Z | jeweils unabhängig voneinander H oder F, |
| $Y^1$ | F, Cl, NCS, $CF_3$, $OCF_3$ oder $OCF_2H$, |
| m | 1 oder 2, |
| n | 1, 2 oder 3, |
| o | 0 oder 1 bedeuten, |

mit der Maßgabe, daß bei $Y^1$ gleich F, Cl oder NCS m 2 oder n 2 oder 3 bedeutet,
oder eine Verbindung der Formel IV ist

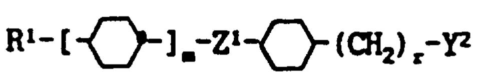

(IV)

worin $R^1$ und m die für die Formel I angegebene Bedeutung besitzen und

$Z^1$ $-(CH_2)_4-$, $-(CH_2)_3-O-$ oder $-CH=CH-CH_2CH_2-$
$Y^2$ $CF_3$, $OCF_3$ oder $-CH=CH-CF_3$ und
r 0 bis 7 bedeuten,

oder daß

(b) mindestens eine Komponente aus Gruppe (B) eine Verbindung der Formel V

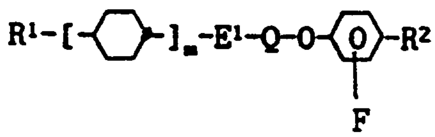

(V)

worin $R^1$, $E^1$, Q und m die für die Formel I angegebene Bedeutung besitzen und

$R^2$ einen Alkyl- oder Alkenylrest mit bis zu 16 C-Atomen, wobei eine oder zwei nicht benachbarte $CH_2$-Gruppen dieser Reste durch -O-, -S-, -CO-O- oder -O-CO- ersetzt sein können,

oder eine Verbindung der Formel VI ist,

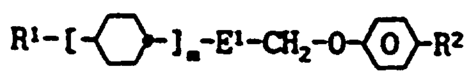

VI

worin $R^1$, $R^2$, $E^1$ und m die für die Formel V angegebene Bedeutung besitzen.

**2.** Nematische flüssigkristalline Zusammensetzung nach Anspruch 1, dadurch gekennzeichnet, daß in den Verbindungen der Formeln I, II, V und VI
$E^1$ $-CH_2CH_2-$
und in den Verbindungen der Formel III
$E^2$ $-(CH_2)_n-$ bedeutet.

**3.** Flüssigkristalline Zusammensetzung nach Anspruch 2, dadurch gekennzeichnet, daß mindestens eine Komponente aus Gruppe (A) aus den Verbindungen der Formeln Ia bis Ih

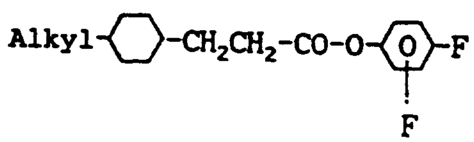

Ia

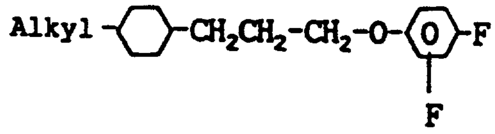

Ib

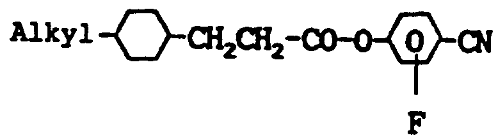

Ic

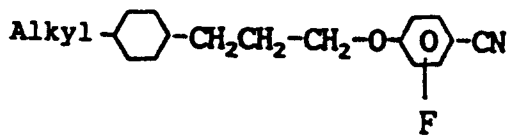

Id

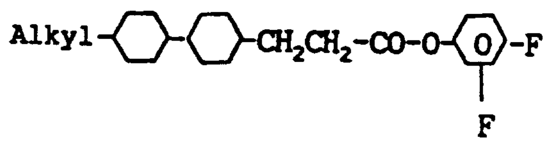

Ie

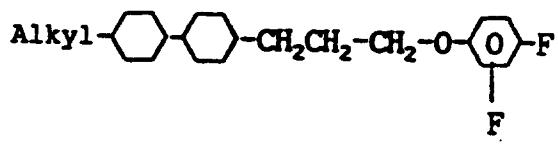

If

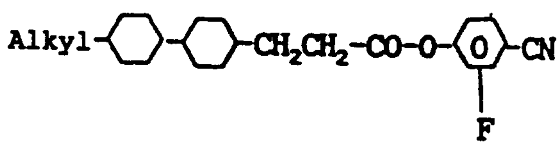

Ig

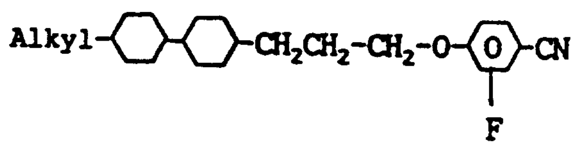

Ih

ausgewählt ist, worin Alkyl einen Alkylrest mit bis zu 16 C-Atomen bedeutet.

**4.** Flüssigkristalline Zusammensetzung nach Anspruch 2, dadurch gekennzeichnet, daß mindestens eine Komponente aus Gruppe (A) aus den Verbindungen der Formeln IIa bis IId:

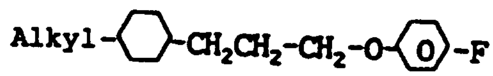

IIa

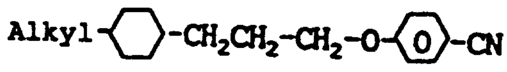

IIb

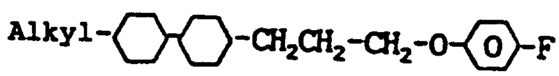

IIc

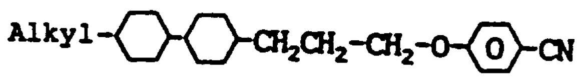

IId

ausgewählt ist.

5. Flüssigkristalline Zusammensetzung nach Anspruch 2, dadurch gekennzeichnet, daß mindestens eine Komponente aus Gruppe (B) aus den Verbindungen der Formeln Va bis Vd oder VIa bis VIb

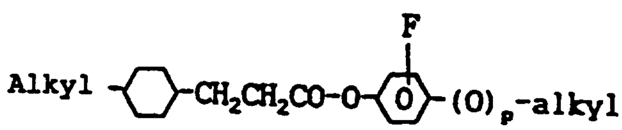

Va

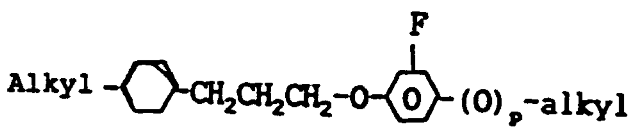

Vb

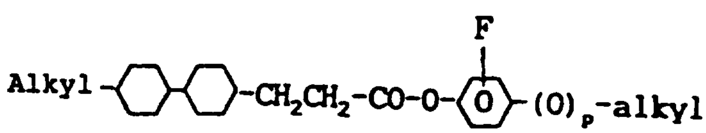

Vc

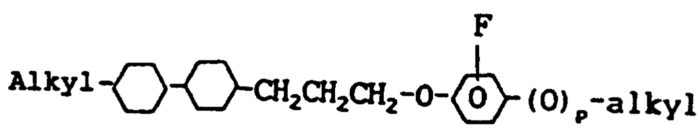

Vd

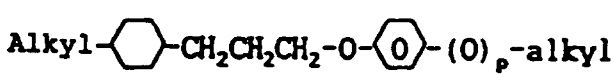

VIa

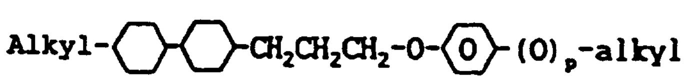

VIb

ausgewählt ist, worin p 0 oder 1 bedeutet.

6. Flüssigkristalline Zusammensetzung nach mindestens einem der Ansprüche 1 bis 5, dadurch gekennzeichnet, daß die dielektrische Anisotropie ($\Delta\epsilon$) der Gruppe A größer als +6 ist.

**7.** Flüssigkristalline Zusammensetzung nach mindestens einem der Ansprüche 1 bis 6, dadurch gekennzeichnet, daß die dielektrische Anisotropie ($\Delta\epsilon$) der Gruppe B -4 bis +3 beträgt.

**8.** Verdrillte nematische Zelle, enthaltend eine flüssigkristalline Zusammensetzung nach mindestens einem der Ansprüche 1 bis 7.

**9.** Zelle nach Anspruch 8, dadurch gekennzeichnet, daß der Verdrillungswinkel zwischen 160 und 300° liegt.

**10.** Cyclohexanderivate der Formel III, ausgewählt aus den Formeln IIIa bis IIIl

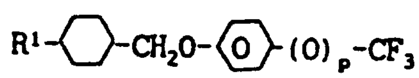

IIIa

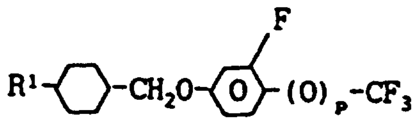

IIIb

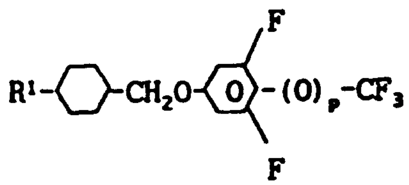

IIIc

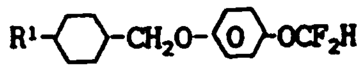

IIId

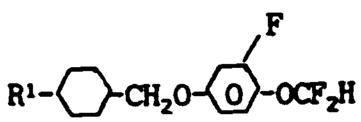

IIIe

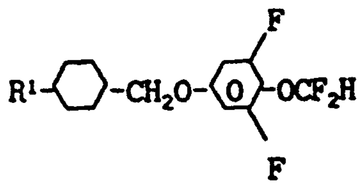

IIIf

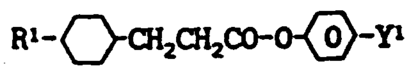

IIIg

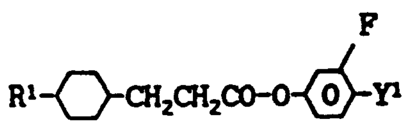

IIIh

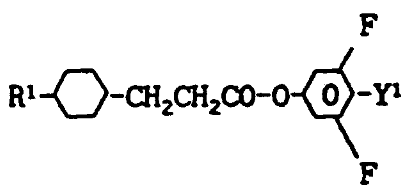

IIIi

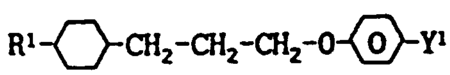

IIIj

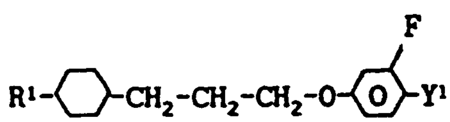

IIIk

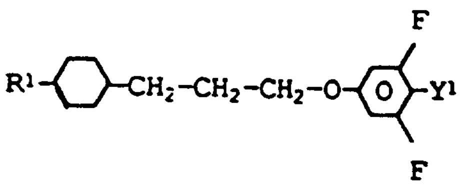

IIIl

worin $R^1$ und $Y^1$ die für die Formel III des Anspruchs 1 angegebene Bedeutung besitzen und p 0 oder 1 ist, mit der Maßgabe, daß

a) die Verbindungen der Formel IIIj mit $Y^1$ gleich F, Cl oder NCS und

b) die Verbindungen der Formel IIIk mit $Y^1$ gleich F ausgenommen sind.

**11.** Cyclohexanderivate der Formel III, ausgewählt aus den Formeln IIIm bis IIIu

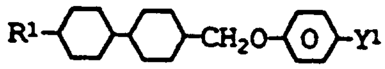

IIIm

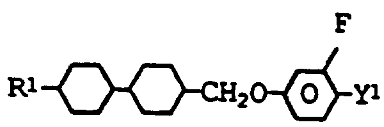

IIIn

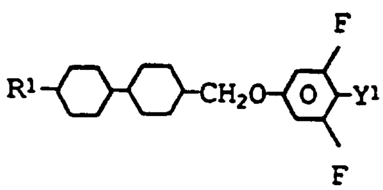

IIIo

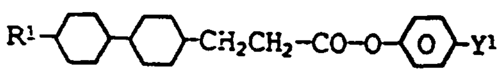

IIIp

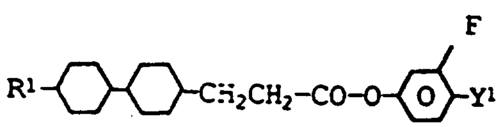

IIIq

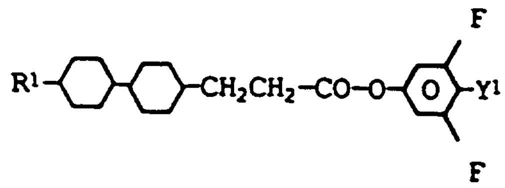

IIIr

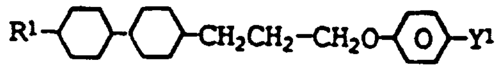

IIIs

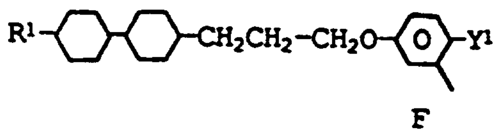

IIIt

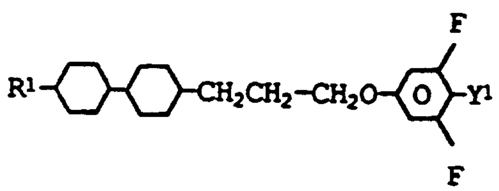

IIIu,

worin $R^1$ und $Y^1$ die für die Formel III des Anspruchs 1 angegebene Bedeutung besitzen, mit der Maßgabe, daß

c) die Verbindungen der Formel IIIs und IIIt mit $Y^1$ gleich F ausgenommen sind.

**12.** Flüssigkristallines Medium nach Anspruch 1, bei dem es sich um eine Mischung aus mindestens zwei Verbindungen handelt, dadurch gekennzeichnet, daß mindestens eine Verbindung ein Cyclohexanderivat der Formel III ist,

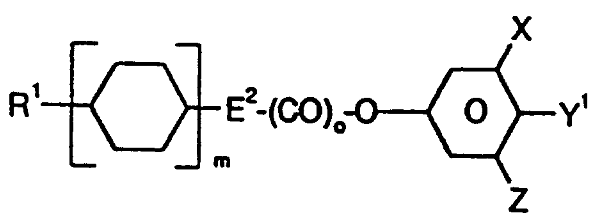

(III)

worin $R^1$, $E^2$, X, Z, $Y^1$, m, n und o die im Anspruch 1 angegebene Bedeutung besitzen.

**13.** Flüssigkristallines Medium nach Anspruch 1, bei dem es sich um eine Mischung aus mindestens zwei Komponenten handelt, dadurch gekennzeichnet, daß mindestens eine Komponente eine Verbindung der Formel IV ist

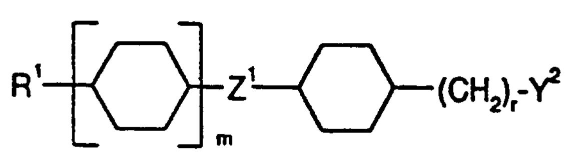

(IV)

worin $R^1$, m, $Z^1$, $Y^2$ und r die im Anspruch 1 angegebene Bedeutung besitzen.

## Revendications

1. Composition cristalline liquide nématique comprenant un groupe (A) avec un ou plusieurs constituants présentant une anisotropie diélectrique ($\Delta\epsilon$) positive et un groupe (B) avec un ou plusieurs constituants présentant une anisotropie diélectrique neutre, caractérisée en ce que
   (a) au moins un constituant du groupe (A) est un composé de formule I

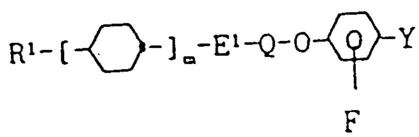

(I)

dans laquelle

- $R^1$ est un résidu alkyle ou alcényle, chacun possédant au plus 16 atomes de C, dans lequel un ou deux groupes $CH_2$ non adjacents de ces résidus peuvent être remplacés par -O-, -S-, -CO-O- ou -O-CO-,
- $E^1$ est $-CH_2CH_2-$ ou -CH = CH-,
- Q est -CO- ou $-CH_2-$,
- Y est un atome d'halogène ou un groupe CN, et
- m est égal à 1 ou 2,

ou un composé de formule II

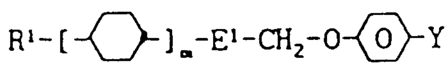

(II)

dans laquelle $R^1$, $E^1$, Y et m ont les significations données pour la formule I,
ou un composé de formule III

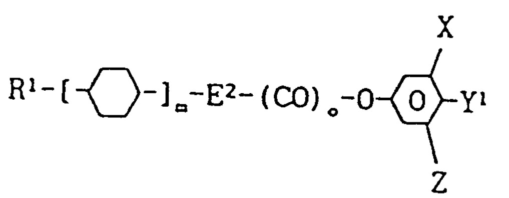

(III)

dans laquelle

- $R^1$ est un résidu alkyle ou alcényle, chacun possédant au plus 16 atomes de C, dans lequel un ou deux groupes $CH_2$ non adjacents de ces résidus peuvent être remplacés par -O-, -S-, -CO-O- ou -O-CO,
- $E^2$ est $-(CH_2)_n$ ou $-CH=CH-(CH_2)_{n-2}-$,
- X et Z sont chacun, indépendamment, H ou F,
- $Y^1$ est F, Cl, NCS, $CF_3$, $OCF_3$ ou $OCF_2H$,
- m est égal à 1 ou 2,
- n est égal à 1, 2 ou 3,
- o est égal à 0 ou 1,

à condition que m soit égal à 2 ou que n soit égal à 2 ou 3 dans le cas où $Y^1$ est F, Cl ou NCS, ou un composé de formule IV

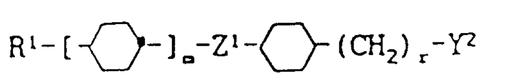

(IV)

dans laquelle $R^1$ et m ont les significations données pour la formule I, et

- $Z^1$ est $-(CH_2)_4-$, $-(CH_2)_3-O-$ ou $-CH=CH-CH_2CH_2-$,
- $Y^2$ est $CF_3$, $OCF_3$ ou $-CH=CH-CF_3$, et

r a une valeur de 0 à 7,

ou en ce que

(b) au moins un constituant du groupe (B) est un composé de formule V

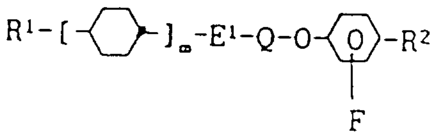

(V)

dans laquelle $R^1$, $E^1$, Q et m ont les significations données pour la formule I, et

$R^2$ est un résidu alkyle ou alcényle possédant au plus 16 atomes de C, dans lequel un ou deux groupes $CH_2$ non adjacents de ces résidus peuvent être remplacés par -O-, -S-, -CO-O- ou -O-CO-,

ou un composé de formule VI

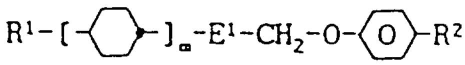

VI

dans laquelle $R^1$, $R^2$, $E^1$ et m ont les significations données pour la formule V.

**2.** Composition cristalline liquide nématique selon la revendication 1, caractérisée en ce que $E^1$ représente $-CH_2CH_2-$, dans les composés de formules I, II, V et VI, et $E^2$ représente $-(CH_2)_n-$, dans les composés de formule III.

**3.** Composition cristalline liquide selon la revendication 2, caractérisée en ce qu'au moins un constituant du groupe (A) est choisi parmi les composés de formules Ia à In

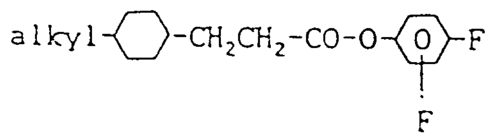

Ia

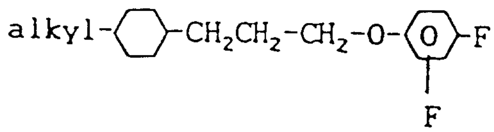

Ib

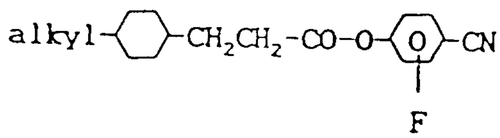

Ic

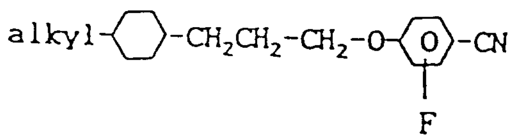

Id

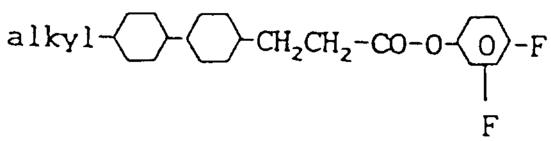

Ie

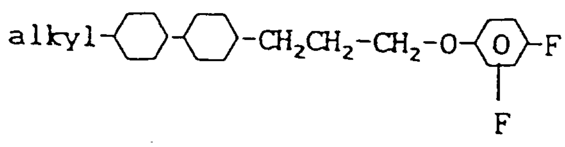

If

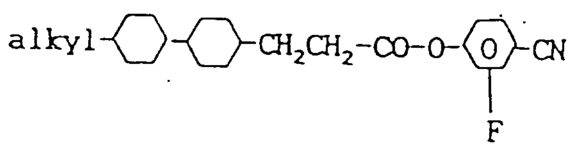

Ig

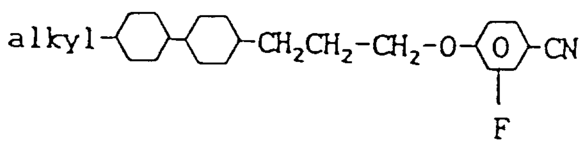

Ih

dans lesquelles alkyl représente un résidu alkyle comportant jusqu'à 16 atomes de C.

**4.** Composition cristalline liquide selon la revendication 2, caractérisée en ce qu'au moins un constituant du groupe (A) est choisi parmi les composés de formules IIa à IId:

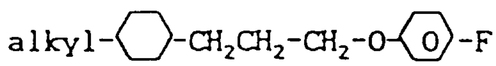 IIa

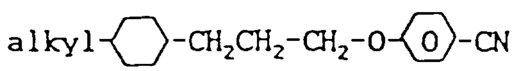 IIb

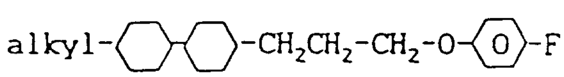 IIc

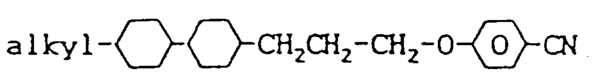 IId

**5.** Composition cristalline liquide selon la revendication 2, caractérisée en ce qu'au moins un constituant du groupe (B) est choisi parmi les composés de formules Va à Vd ou VIa à VIb:

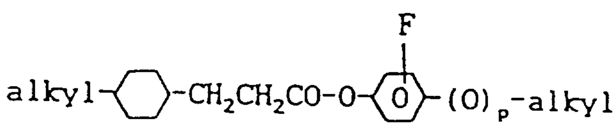 Va

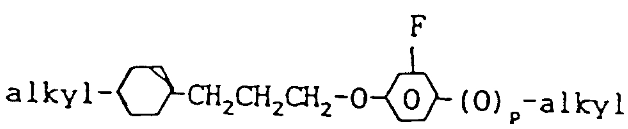 Vb

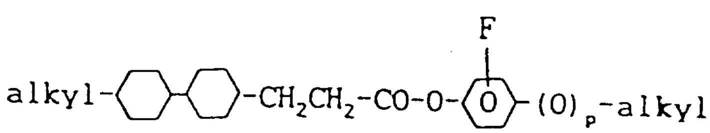 Vc

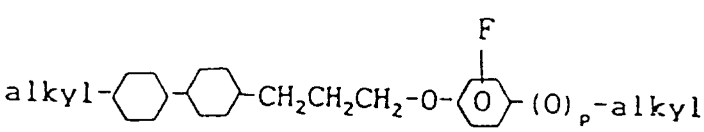 Vd

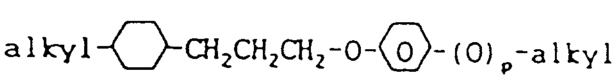 VIa

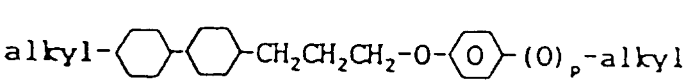 VIb

dans lesquelles p est égal à 0 ou 1.

**6.** Composition cristalline liquide selon l'une au moins des revendications 1 à 5, caractérisée en ce que l'anisotropie diélectrique ($\Delta\epsilon$) du groupe A est supérieure à +6.

7. Composition cristalline liquide selon l'une au moins des revendications 1 à 6, caractérisée en ce que l'anisotropie diélectrique (Δε) du groupe B est de -4 à +3.

8. Cellule nématique tordue contenant une composition cristalline liquide selon l'une au moins des revendications 1 à 7.

9. Cellule selon la revendication 8, caractérisée en ce que l'angle de torsion est compris entre 160 et 300°.

10. Dérivés de cyclohexane de formule III, choisis parmi les formules IIIa à IIII

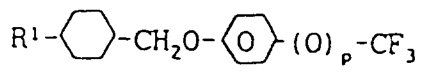

IIIa

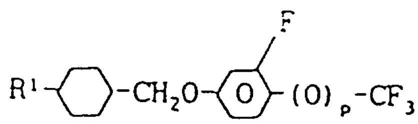

IIIb

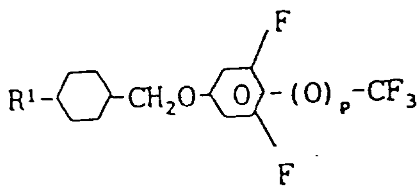

IIIc

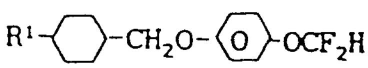

IIId

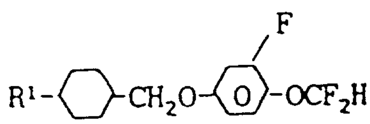

IIIe

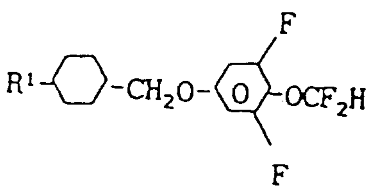

IIIf

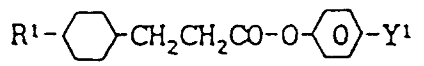

IIIg

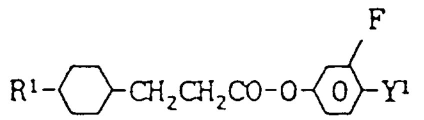

IIIh

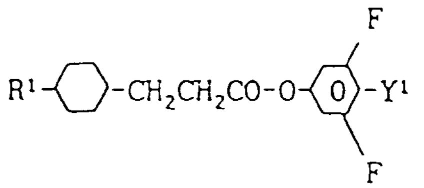

IIIi

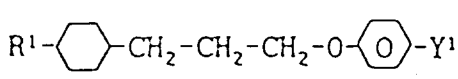

IIIj

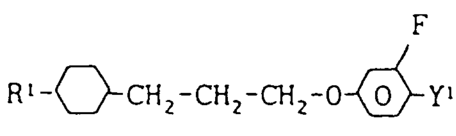

IIIk

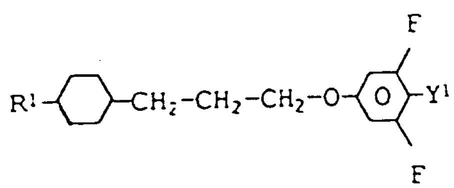

IIIl

dans lesquelles $R^1$ et $Y^1$ ont les significations données pour la formule III de la revendication 1, et p est égal à 0 ou 1, à condition que

a) les composés de formule IIIj, dans laquelle $Y^1$ est F, Cl ou NCS, soient exclus, et que

b) les composés de formule IIIk, dans laquelle $Y^1$ est F, soient exclus.

**11.** Dérivés de cyclohexane de formule III, choisis parmi les formules IIIm à IIIu

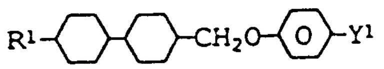

IIIm

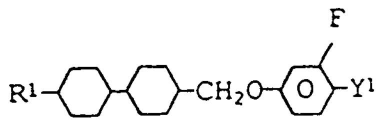

IIIn

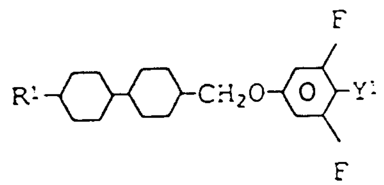

IIIo

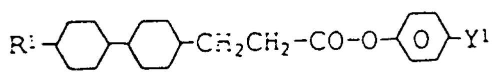

IIIp

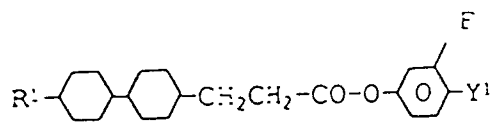

IIIq

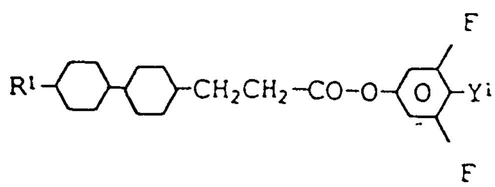

IIIr

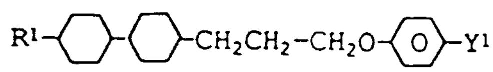

IIIs

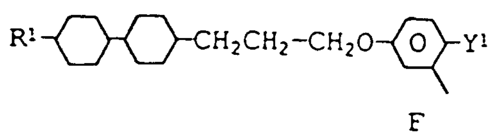

IIIt

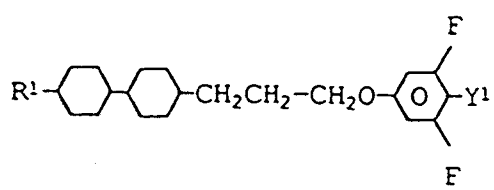

IIIu

dans lesquelles $R^1$ et $Y^1$ ont les significations données pour la formule III de la revendication 1, à condition que

c) les composés de formules IIIs et IIIt, dans lesquelles $Y^1$ est F, soient exclus.

**12.** Milieu cristallin liquide selon la revendication 1, qui est un mélange d'au moins deux composés, caractérisé en ce qu'au moins un des composés est un dérivé de cyclohexane de formule III

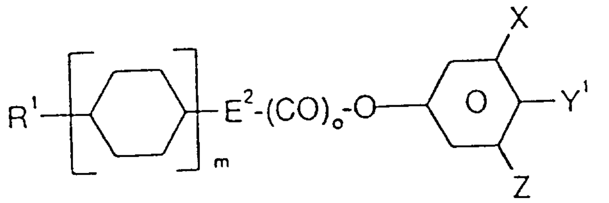

(III)

dans laquelle $R^1$, $E^2$, X, Z, $Y^1$, m, n et o ont les significations données dans la revendication 1.

**13.** Milieu cristallin liquide selon la revendication 1, qui est un mélange d'au moins deux composants, caractérisé en ce qu'au moins un des composants est un composé de formule IV

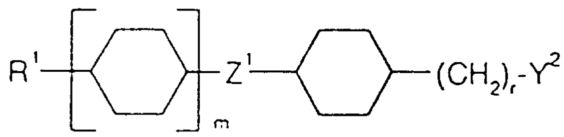

(IV)

dans laquelle $R^1$, m, $Z^1$, $Y^2$ et r ont les significations données dans la revendication 1.